# EUROPEAN PATENT APPLICATION

(11) **EP 2 392 680 A1**
(43) Date of publication of application: **07.12.2011**
(21) Application number: 10164943.2
(22) Date of filing: 04.06.2010
(51) Int. Cl.: C12Q 1/70

(54) **Method for specific detection of classical swine fever virus**

(71) Applicant: Labor Diagnostik GmbH Leipzig, 04103 Leipzig (DE)
(72) Inventor: Hoffmann, Bernd, 17498 Neuenkirchen (DE); Leifer, Immanuel, 17495 Grosskiesow (DE); Beer, Martin, 17498 Neuenkirchen (DE); Gaunitz, Christine, 04416 Markkleeberg (DE)
(74) Representative: Vossius & Partner

(57) **Abstract**

The present invention relates to a method for determining the presence of a classical swine fewer virus (CSFV) in a subject comprising the detection of the NS5A region of at least one classical swine fewer virus (CSFV) genome in a sample derived from said subject. The invention further relates to a kit for determining the presence of a classical swine fewer virus (CSFV) and to nucleic acids as well as to the use of the kit and/or nucleic acids in said method.

## Description

### Background of the invention

Classical swine fever (CSF) is a highly contagious and often fatal infectious disease in domestic pigs and wild boar. Outbreaks in industrialized pig productions are controlled by sanitary measures and large scale culling, and cause significant economic losses (Edwards, S., Fukusho, A., Lefevre, P.C., Lipowski, A., Pejsak, Z., Roehe, P., Westergaard, J., 2000. Classical swine fever: the global situation. Vet. Microbiol. 73 (2-3), 103-119; Fauquet C.M. et al. 2005. Virus Taxonomy, VIIIth Report of the International Committee on Taxonomy of Viruses. Elsevier / Academic press, 2005; Vandeputte, J. and Chappuis, G., 1999. Classical swine fever: the European experience and a guide for infected areas. Rev. Sci. Tech. 18 (3), 638-647). Outbreaks of CSF have to be reported to the OIE (Office International des Epizooties) and the disease is controlled within the European Union (EU) by a stamping-out policy without prophylactic vaccination. Despite sustained eradication efforts, outbreaks of CSF still occur regularly in European domestic pigs.

CSF virus ("CSFV") is a member of the genus Pestivirus of the familiy Flaviviridae, and is closely related to bovine viral diarrhoea virus ("BVDV") and border disease virus ("BDV") (Fauquet, C.M. and Fargette, D., 2005. International Committee on Taxonomy of Viruses and the 3,142 unassigned species. Virol. J. 2, 64). The natural hosts for BVDV and BDV are cattle and sheep, respectively, but both viruses also naturally infect pigs. It is important to differentiate between CSFV and BVDV or BDV.

Pestiviruses are small, positive-sense, single-stranded RNA viruses with a genome of about 12.3 kb (Meyers, G. and Thiel, H.J., 1996. Molecular characterization of pestiviruses. Adv. Virus Res. 47, 53-118). The genome comprises one open reading frame encoding a single polyprotein of 3.898 amino acids that is flanked by a 5'terminal and a 3'terminal untranslated region (UTR). The polyprotein is co-and post-translationally processed by viral and cellular proteases (Meyers, G. and Thiel, H.J., 1996. Molecular characterization of pestiviruses. Adv. Virus Res. 47, 53-118). It is cleaved into four structural proteins (C, ERNS, E1 and E2) and seven non-structural proteins (Npro, p7, NS2, NS3, NS4A, NS4B, NS5A and NS5B) (Tautz, N., Elbers, K., Stoll, D., Meyers, G., Thiel H.J., 1997. Serine protease of pestiviruses: determination of cleavage sites. J. Virol. 71(7), 5415-22).

For effective control and prevention of virus spread during CSF outbreaks, the fast, sensitive and reliable detection of CSFV is of particular importance. CSFV can be detected by virus isolation, antigen ELISA, gel-based RT-PCR and real-time RT-PCR protocols (Aguero, M., Fernandez, J., Romero, L.J., Zamora, M.J., Sanchez, C., Belak, S., Arias, M., Sanchez-Vizcaino, J.M., 2004. A highly sensitive and specific gel-based multiplex RT-PCR assay for the simultaneous and differential diagnosis of African swine fever and Classical swine fever in clinical samples. Vet. Res. 35 (5), 551-563; Hergarten, G., Hurter, K.P., Hess, R.G., 2001. [Detection of infection with classical swine fever virus in wild boar: a comparison of different laboratory diagnostic methods.]. Dtsch. Tierarztl. Wochenschr. 108 (2), 51-54).

Different real-time RT-PCR protocols are available and used in the diagnostics laboratories (Barlic-Maganja, D. and Grom, J., 2001. Highly sensitive one-tube RT-PCR and microplate hybridisation assay for the detection and for the discrimination of classical swine fever virus from other pestiviruses. J. Virol. Methods 95 (1-2), 101-110; Hoffmann, B., Beer, M., Schelp, C., Schirrmeier, H., Depner, K., 2005. Validation of a real-time RT-PCR assay for sensitive and specific detection of classical swine fever. J. Virol. Methods 130 (1-2), 36-44; Hoffmann, B., Depner, K., Schirrmeier, H., Beer, M., 2006. A universal heterologous internal control system for duplex real-time RT-PCR assays used in a detection system for pestiviruses. J. Virol. Methods 136 (1-2), 200-209; Leifer, I., Depner, K., Blome, S., Le Potier, M.F., Le, D.M., Beer, M., Hoffmann, B., 2009a. Differentiation of C-strain "Riems" or CP7_E2alf vaccinated animals from animals infected by classical swine fever virus field strains using real-time RT-PCR. J. Virol. Methods 158 (1-2), 114-122; Zhao, J.J., Cheng, D., Li, N., Sun, Y., Shi, Z., Zhu, Q.H., Tu, C., Tong, G.Z., Qiu, H.J., 2008. Evaluation of a multiplex real-time RT-PCR for quantitative and differential detection of wild-type viruses and C-strain vaccine of Classical swine fever virus. Vet. Microbiol. 126 (1-3), 1-10). All established protocols use the conserved 5' UTR as template for CSFV field strain detection. Recently a C-strain specific protocol was described that uses the E^{RNS} genome region for specific detection of CSF C-strain "Riems" vaccine virus (Leifer, I., Depner, K., Blome, S., Le Potier, M.F., Le, D.M., Beer, M., Hoffmann, B., 2009a. Differentiation of C-strain "Riems" or CP7_E2alf vaccinated animals from animals infected by classical swine fever virus field strains using real-time RT-PCR. J. Virol. Methods 158 (1-2), 114-122). In situations with high throughput of positive sample material, amplicon contamination from the real-time RT-PCR itself or from other RT-PCR applications targeting the same genome region like sequence analysis can interfere with CSFV diagnostics. Thus, there is a need for a further specific method for detection of CSFV. The new method has to be compatible with the detection of reference genes, i.e. the detection of a reference gene does interfere with the new detection method.

The inventors unexpectedly found that CSFV can be specifically detected by detection of the NS5A region or parts thereof. Thus, the present invention provides a new method for specific CSFV detection. Furthermore, the inventors were able to detect strains of Classical Swine Fever Virus (CSFV) of which the NS5A region was unknown (SEQ ID NO. 37 to 41). This unambiguously shows that the method according to the present invention provides a method by which all CSFV strains can be detected while being very specific for CSFV.

### Description of the invention

The present invention relates on a method for determining the presence of a classical swine fewer virus (CSFV) in a subject comprising the following step:
- detection of the NS5A region or parts thereof of at least one classical swine fewer virus (CSFV) genome in a sample derived from said subject;
wherein the detection of the NS5A region or parts thereof of the at least one classical swine fewer virus (CSFV) genome is attributed to the presence of a classical swine fewer virus (CSFV) in said subject.

A "Sample" in the meaning of the invention can be all biological tissues and all fluids such as lymph, urine, cerebral fluid, blood, saliva, serum, faeces, plasma, cell culture supernatant. Tissues may be, e.g. organs tissue like spleen, kidney, tonsil, lymp node, epithelium tissue, connective tissue such as bone or blood, muscle tissue such as visceral or smooth muscle and skeletal muscle and, nervous tissue. Tissue may also be selected from the group consisting of bone marrow, cartilage, skin, mucosa and hair. The sample is collected from the patient or subject in which the presence of CSFV shall be determined according to the invention. Where appropriate, as for instance in the case of solid samples, the sample may need to be solubilised, homogenized, or extracted with a solvent prior to use in the present invention in order to obtain a liquid sample. A liquid sample hereby may be a solution or suspension. Liquid samples may be subjected to one or more pre-treatments prior to use in the present invention. Such pre-treatments include, but are not limited to dilution, filtration, centrifugation, concentration, sedimentation, precipitation, dialysis. Pre-treatments may also include the addition of chemical or biochemical substances to the solution, such as acids, bases, buffers, salts, solvents, reactive dyes, detergents, emulsifiers, chelators.

By NS5A region any the region of the genome of a classical swine fewer virus is meant which shows at least 70% sequence identity to the region of nucleotides 8423 to 9913 of SEQ ID NO. 1, preferably at least 80% sequence identity, more preferably at least 90% sequence identity, even more preferably at least 95% sequence identity (including but not limited to at least 96% sequence identity, at least 97% sequence identity, at least 98% sequence identity, at least 99% sequence identity, at least 99.5% sequence identity and 100% sequence identity). The determination of percent identity between two sequences may accomplished by different methods and algorithms known by those skilled in the art, e.g. by using the mathematical algorithm of Karlin and Altschul (1993) Proc. Natl. Acad. Sci. USA 90: 58735877. Such an algorithm is incorporated into the BLASTN and BLAST programs of Altschul et al. (1990) J. Mol. Biol. 215: 403-410. BLAST nucleotide searches are performed with the BLASTN program, score = 100, word length = 12, to obtain nucleotide sequences showing a given sequence identity to the region of genomes of classical swine fewer viruses (CSFV) recited herein. To obtain gapped alignments for comparative purposes, Gapped BLAST is utilized as described in Altschul et al. (1997) Nucleic Acids Res. 25: 3389-3402. When utilizing BLAST and Gapped BLAST programs, the default parameters of the respective programs are used.

In a further preferred embodiment the NS5A region of the genome of the at least one classical swine fewer virus is the NS5A region of at least one of the genomes of the classical swine fewer viruses (CSFV) strain selected from the group consisting of the known strains AY072924 "Paderborn", EU490425 '"Thiverval" ,EU497410 "JL1(06)", AY775178 "Shimen/HVRI", AY646427 "IL/94/TWN", AY805221 "C/HVRI", DQ127910 "SWH", X8793 "Alfort/187", J04358 "Alfort/Tueb", X96550 "CAP", AY578688 "RUCSFPLUM", AY578687 "BRESCIAX", AY55439796 "TD", AY663656 "China", AY259122 "Riems", AF326963 "Eystrup" (SEQ ID NO. 1), AY382481 "China vacc.", AY568569 "CH/01/TWN", AY367767 "GXWZ02", AF531433 "HCLV", AF407339 "strain 39", AF333000 "cF114", AF099102 "Russian vacc.", AF092448 "Shimen", AF091507 "HCLV", AF091661 "Brescia", U90951 "Alfort A19", U45478 "Glentorf", U45477 "Riems", as well as the strains with newly identified and sequenced NS5A region CSFV Koslov, CSFV Hennef, CSFV Borken, CSFV Roesrath, and CSFV Bergen.

In one embodiment of the invention the NS5A region is detected by detecting one or more of the sequences selected from the group consisting of SEQ ID NOs 8, 9, 10, 11, 12, 13, 14, 15, 16, 17, 18, 19, 20, 21, 22, 23, 24, 25, 26, 27, 28 ,29, 30, 31, 32, 33, 34, 35, 36, 37, 38, 39, 40, and 41, or fragments thereof. In a further embodiment said fragment comprises at least the sequence according to SEQ ID NO. 2.

Methods and means for the specific detection of sequences within a sample comprising a mixture of nucleic acid is well known by those skilled in the art. Such method may comprise sequencing methods (including Sanger method (Sanger, F. et al. (1977): DNA sequencing with chain-terminating inhibitors. Proc. Natl. Acad. Sciences US 74, 5463-5467), shot gun- and pyrosequencing (Ronaghi, M (2001): Pyrosequencing sheds light on DNA sequencing. Genome Research 11, 3-11; Bankier AT (2001) Shotgun DNA sequencing. Methods Mol. Biol. 167, 89-100, GS FLX Pyrosequencing (Droege, M. Hill, B. (2008): The Genome Sequencer FLX System- longer reads, more applications, straight forward bioinformatics and more complete data sets. J. Biotechnol. 136, 3-10) and other next-generation sequencing technologies like Illumina/Solexa, ABI/Solid (Morozova, O., Marra, M.A. (2008): Applications of next-generation sequencing technologies in functional genomics. Genomics 92, 255-64), *in-situ* hybridisation, Northern blot, polymerase chain reaction (PCR), quantitative PCR (qPCR), real-time PCR, reverse transcriptase PCR (RT-PCR), real-time RT-PCR, Microarray analysis.

The detection of the NS5A region in one embodiment comprises the use of a nucleic acid probe hybridizing to the NS5A region of at least one classical swine fewer virus (CSFV) genome and/or to a nucleic acid complementary to the at least one NS5A region of the at least one classical swine fewer virus (CSFV) genome.

"Nucleic acid", in accordance with the present invention, includes DNA, such as cDNA or genomic DNA, and RNA. It is understood that the term "RNA" as used herein comprises all forms of RNA including mRNA as well as genomic RNA (gRNA) of viruses. Preferably, embodiments reciting "RNA" are directed to gRNA of the virus. In connection with DNA the rules for nomenclature of the International Union of Pure and Applied Chemistry (IUPAC) are used which are as follows: A is adenine, C is cytosine, G is guanine, T is thymine, R is G or A , Y is T or C, K is G or T, M is A or C, S is G or C, W is A or T, B is G or T or C (all but A), D is G or A or T (all but C), H is A or C or T (all but G), V is G or C or A (all but T), N is A or G or C or T (any). These symbols are also valid for RNA, although U replaces T (for uracil rather than thymine).

"Nucleic acid probe", in accordance with the present invention is an oligonucleotide, nucleic acid or a fragment thereof that specifically hybridizes with a target sequence in a nucleic acid molecule, e.g. gRNA of a CSFV or cDNA or amplification products thereof. Thus, a nucleic acid probe according to the present invention is an oligonucleotide, nucleic acid or a fragment thereof, which is substantially complementary to a specific target sequence. A substantially complementary nucleic acid may have a sequence completely complementary to the target sequence. However, also mismatches of one or more nucleotides may be allowed, so long as the nucleic acid probe can specifically hybridize to the target sequence(s) under stringent conditions. For example, the nucleic acid probe of the present invention include oligonucleotides that have a sequence identity to the target sequence of at least 70%, preferably at least 80%, more preferably at least 90%, even more preferably at least 95% sequence identity (including but not limited to at least 96% sequence identity, at least 97% sequence identity, at least 98% sequence identity, at least 99% sequence identity, at least 99.5% sequence identity and 100% sequence identity). It may also be desirable that a nucleic acid probe binds to a variety of sequences which differ by one or more nucleotides. A way to encounter this problem is the use of so called degenerated nucleic acid probes. Degenerated nucleic acid probes are mixtures of oligonucleotides which differ in sequence at one or more positions. The oligonucleotides of degenerated nucleic acid probes may differ in their length or may all have the same length. In one embodiment of the present invention the nucleic acid probe is a degenerated nucleic acid probe and hybridizes to NS5A regions of essentially all classical swine fewer viruses (CSFV) genomes.

In one embodiment of the present invention nucleic acid probes (for CSFV as well as for reference nucleic acids) have a length of 7 to 40 nucleotides, preferably 12 to 30 nucleotides, more preferably 15 to 20 nucleotides, even more preferably the nucleic acid probe has a length of 16 nucleotides.

In a further embodiment the nucleic acid probe hybridizes to a nucleic acid having at least 70% sequence identity to the sequence of nucleotides 8423 to 9913 according to SEQ ID NO. 1, preferably at least 80% sequence identity, more preferably at least 90% sequence identity, even more preferably at least 95% sequence identity (including but not limited to at least 96% sequence identity, at least 97% sequence identity, at least 98% sequence identity, at least 99% sequence identity, at least 99.5% sequence identity and 100% sequence identity).

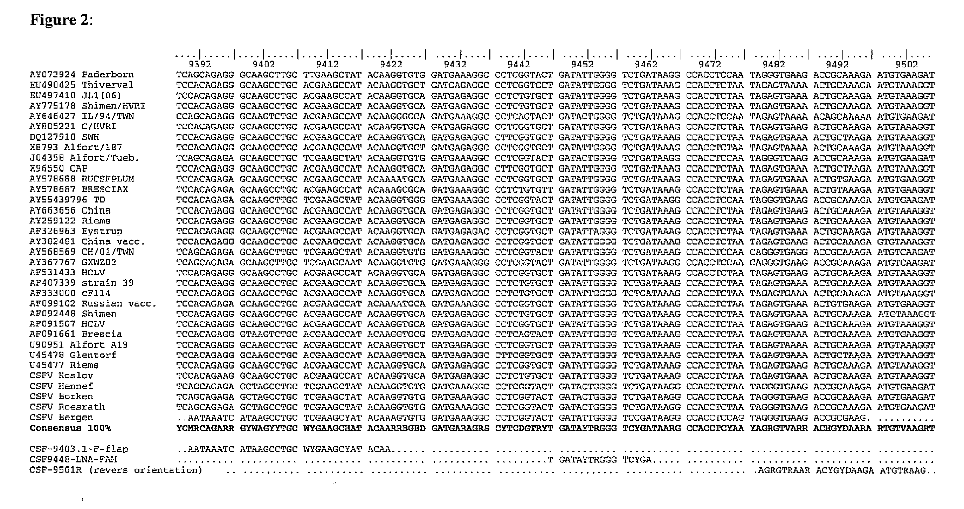

In a preferred embodiment the nucleic acid probe hybridizes to a nucleic acid having a sequence selected from the group consisting of SEQ ID NOs 8, 9, 10, 11, 12, 13, 14, 15, 16, 17, 18, 19, 20, 21, 22, 23, 24, 25, 26, 27, 28 ,29, 30, 31, 32, 33, 34, 35, 36, 37, 38, 39, 40, and 41 (see Figure 2). In a further embodiment the nucleic acid probe hybridizes to a nucleic acid having any of the sequences according to SEQ ID NOs 8, 9, 10, 11, 12, 13, 14, 15, 16, 17, 18, 19, 20, 21, 22, 23, 24, 25, 26, 27, 28 ,29, 30, 31, 32, 33, 34, 35, 36, 37, 38, 39, 40, and 41.

In a further embodiment the nucleic acid probe hybridizes to a nucleic acid having at least 70% sequence identity to the sequence of nucleotides 9400 to 9505 according to SEQ ID NO. 1, preferably at least 80% sequence identity, more preferably at least 90% sequence identity, even more preferably at least 95% sequence identity (including but not limited to at least 96% sequence identity, at least 97% sequence identity, at least 98% sequence identity, at least 99% sequence identity, at least 99.5% sequence identity and 100% sequence identity).

In a further embodiment the nucleic acid probe hybridizes to a nucleic acid having at least 70% sequence identity to the sequence of nucleotides 9440 to 9469 according to SEQ ID NO. 1, preferably at least 80% sequence identity, more preferably at least 90% sequence identity, even more preferably at least 95% sequence identity (including but not limited to at least 96% sequence identity, at least 97% sequence identity, at least 98% sequence identity, at least 99% sequence identity, at least 99.5% sequence identity and 100% sequence identity).

In a further embodiment the nucleic acid probe hybridizes to a nucleic acid having at least 70% sequence identity to the sequence of nucleotides 9447 to 9462 according to SEQ ID NO. 1, preferably at least 80% sequence identity, more preferably at least 90% sequence identity, even more preferably at least 95% sequence identity (including but not limited to at least 96% sequence identity, at least 97% sequence identity, at least 98% sequence identity, at least 99% sequence identity, at least 99.5% sequence identity and 100% sequence identity).

In one embodiment of the present invention the nucleic acid probe has between 16 and 40 nucleotides and comprises the sequence according to SEQ ID NO. 2.

As outlined above it might be desirable that the classical swine fewer virus (CSFV) is detected specifically. Thus in one embodiment of the present invention the nucleic acid probe does not hybridize to a bovine viral diarrhoea virus (BVDV) and does not hybridize to a border disease virus (BDV) under stringent conditions.

The hybridization of a nucleic acid probe to DNA or RNA from a sample is an indication of the presence of the relevant DNA or RNA in the sample. Hybridization conditions are known to those skilled in the art and can be found in Current Protocols in Molecular Biology, John Wiley & Sons, N.Y., 6.3.1-6.3.6, 1991. In one embodiment of the present invention the nucleic acid probe does hybridize to the NS5A region of at least one classical swine fewer virus (CSFV) but does not to nucleic acids from BVDV and BDV or to nucleic acids complementary thereon under stringent conditions. Stringent conditions are defined as equivalent to hybridization in 6X sodium chloride/sodium citrate (SSC) at 45°C, followed by a wash in 0.2 X SSC, 0.1 % SDS at 65°C

In a preferred embodiment the nucleic acid probe does not hybridize to a bovine viral diarrhoea virus (BVDV) and does not hybridize to a border disease virus (BDV). The skilled artisan will recognize that it is desirable that the nucleic acid probe does not hybridize to BVDV and BDV under stringent conditions. However, the skilled artisan is aware of the fact that under certain circumstances unspecific hybridization of nucleic acids may occur und non stringent conditions and/or due to high concentration of "unspecific" target nucleic acids. Thus, the nucleic acid probe according to the invention might hybridize unspecific to the nucleic acid of BVDV or BDV under unfavourable conditions, e.g. high concentrations of nucleic acids of BVDV and/or BDV as they might occur in e.g. under cell culture conditions. Nevertheless the nucleic acid probe according to the invention does not hybridize to nucleic acids of BVDV or BDV under conditions of the method according to the invention, i.e. with concentrations of nucleic acids BVDV and/or BDV as they occur in samples of subjects or in concentrations of nucleic acids of BVDV and/or BDV as they occur after amplification of the NS5A region of a CSFV or fragments thereof with specific CSFV amplification primers according to the present invention.

Further included are nucleic acid mimicking molecules known in the art such as synthetic or semisynthetic derivatives of DNA or RNA and mixed polymers, both sense and antisense strands. They may contain additional non-natural or derivatized nucleotide bases, as will be readily appreciated by those skilled in the art. Such nucleic acid mimicking molecules or nucleic acid derivatives according to the invention include peptide nucleic acid, phosphorothioate nucleic acid, phosphoramidate nucleic acid, 2'-O-methoxyethyl ribonucleic acid, morpholino nucleic acid, hexitol nucleic acid (HNA), 3'-minor groove binder oligodeoxynucleotide conjugates (MGB-ODN) and locked nucleic acid (LNA) (see, for example, Braasch and Corey, Chemistry & Biology 8, 1-7 (2001); and Kutyavin, Afonina, Mills et al., Nucleic Acid Research 28(2), 655-661 (2000)). LNA is an RNA derivative in which the ribose ring is constrained by a methylene linkage between the 2'-oxygen and the 4'-carbon.

In one embodiment of the present invention the nucleic acid probe comprises at least one modification to increase the stability of the probe-target hybrid. Such modifications are known by those skilled in the art. In one embodiment of the present invention the oligonucleotides of the nucleic acid probe comprise at least one modification selected from the group consisting of locked nucleic acids (LNA), peptide nucleic acid (PNA) and 3'-minor groove binder (MGB).

For the purposes of the present invention, a peptide nucleic acid (PNA) is a polyamide type of DNA analogue with an amide backbone in place of the sugar-phosphate backbone of DNA. As a consequence, certain components of DNA, such as phosphorus, phosphorus oxides, or deoxyribose derivatives, are not present in PNAs. As disclosed by Nielsen et al., Science 254:1497 (1991); and Egholm et al., Nature 365:666 (1993), PNAs bind specifically and tightly to complementary DNA strands and are not degraded by nucleases. Furthermore, they are stable under acidic conditions and resistant to proteases (Demidov et al. (1994), Biochem. Pharmacol., 48, 1310-1313). Their electrostatically neutral backbone increases the binding strength to complementary DNA as compared to the stability of the corresponding DNA-DNA duplex (Wittung et al. (1994), Nature 368, 561-563; Ray and Norden (2000), Faseb J., 14, 1041-1060).

For the purposes of the present invention, Locked nucleic acid (LNA) nucleosides are a class of nucleic acid analogues in which the ribose ring is "locked" by a methylene bridge connecting the 2'-O atom and the 4'-C atom. LNA nucleosides contain the common nucleobases (T, C, G, A, U and mC) and are able to form base pairs according to standard Watson-Crick base pairing rules. When incorporated into a DNA oligonucleotide, LNA therefore hybridize with a complementary nucleotide strand more rapid and increases the stability of the resulting duplex. As a result the duplex melting temperature (Tm) may increase by up to 8°C per LNA monomer substitution. (see e.g. Peterson, M. and Wengel, J. (2003) LNA: a versatile tool for therapeutics and genomics. Trends Biotechnol. 21, 74-81).

For purpose of the present invention a 3'-Minor groove binder probe is a nucleic acid probe which is covalently linked to a minor groove binding conjugate at the 3'-end of said nucleic acid probe. Minor groove binding conjugates are well known by those skilled in the art (see e.g. Kutyavin, Afonina, Mills et al., Nucleic Acid Research 28(2), 655-661 (2000)). They may be selected from e.g. DPI₃ and CDPI₃. Furthermore, it will be easily recognized by those skilled in the art that the minor groove binding conjugates may be directly linked to the 3'-end of the nucleic acid probe or may be indirectly linked to the 3'-end of the nucleic acid probe via a linker. Such linkers are well known by the skilled artisan (see e.g. Kutyavin, Afonina, Mills et al., Nucleic Acid Research 28(2), 655-661 (2000)). Examples of such linkers are aminopropyl or alkylamine linker. In one embodiment the minor groove binding conjugate binds to the minor groove formed by the 3' terminal 5 to 6 bp of the nucleic acid probe/target nucleic acid probe duplex.

In fact, PNA, LNA or MGB-nucleic acid probes bind more strongly to DNA than DNA itself does. Because of this, PNA/DNA, LNA/DNA and MGB-nucleic acid probes/DNA duplexes bind under a wider range of stringency conditions than DNA/DNA duplexes, making it easier to perform multiplex hybridization. Smaller probes can be used than with DNA due to the strong binding. In addition, it is more likely that single base mismatches can be determined with PNA/DNA, LNA/DNA and MGB-nucleic acid probes/DNA hybridization because a single mismatch in a PNA/DNA, LNA/DNA and MGB-nucleic acid probes/DNA 15-mer lowers the melting point (Tₘ) by 8°-20° C, vs. 4°-16° C for the DNA/DNA 15-mer duplex. Thereby the stability of the probe-target hybrid is increased. This also improves discrimination between perfect matches and mismatches. PNA, LNA and MGB-nucleic acid probes also permit the hybridisation of DNA samples at low salt or no-salt conditions. As a consequence, the target DNA has fewer secondary structures under hybridisation conditions and is more accessible to probe molecules.

In one embodiment the nucleic acid probe comprises at least at least 4 modifications to increase the stability of the probe-target hybrid, more preferably at least 5, even more preferably 7.

In one embodiment the nucleic acid probe has the sequence of SEQ ID NO. 2. In a further embodiment the nucleic acid probe has the sequence of SEQ ID NO. 2 and has 7 LNA. In a further embodiment the nucleic acid probe comprises at least 4 modifications to increase the stability of the probe-target hybrid, wherein said modifications are at four positions selected from the group consisting of positions 2, 4, 7, 9, 11, 13 and 15 of SEQ ID NO. 2. In a yet further embodiment the nucleic acid probe comprises at least 5 modifications to increase the stability of the probe-target hybrid, wherein said modifications are at five positions selected from the group consisting of positions 2, 4, 7, 9, 11, 13 and 15 of SEQ ID NO. 2. In a more preferred the nucleic acid has the sequence according to SEQ ID NO. 2 and has LNAs at position 2, 4, 7, 9, 11, 13 and 15.

In some cases, the concentration of classical swine fewer virus (CSFV) present in a subject may be below detectable levels. In such case it is desirable to amplify the genome of at least one classical swine fewer virus (CSFV) or fragments thereof. Thus, in one embodiment of the present invention prior to the detection of the NS5A region of the at least one classical swine fewer virus (CSFV) genome said genome of the at least one classical swine fewer virus (CSFV) or parts thereof is amplified. The skilled artisan will unambiguously recognize that the region which has to be detected must be part of the amplified part of the at least one classical swine fewer virus (CSFV) genome, i.e. the region to which the nucleic acid probe specifically hybridizes has to be part of the amplified region.

Nucleic-acid amplification can be accomplished by any of the various nucleic-acid amplification methods known in the art, including but not limited to the polymerase chain reaction (PCR), ligase chain reaction (LCR) (such as in Landegren, et al., "A Ligase-Mediated Gene Detection Technique," Science 241:1077-1080, 1988, or, in Wiedmann, et al., "Ligase Chain Reaction (LCR)--Overview and Applications," PCR Methods and Applications (Cold Spring Harbor Laboratory Press, Cold Spring Harbor Laboratory, NY, 1994) pp. S51-S64.)), transcription-based amplification system (TAS), nucleic acid sequence based amplification (NASBA), rolling circle amplification (RCA), transcription-mediated amplification (TMA), self-sustaining sequence replication (3SR), isothermal amplification (such as in Walker, et al., "Strand displacement amplification--an isothermal, in vitro DNA amplification technique," Nucleic Acids Res. 20(7):1691-6 (1992)) and Qβ amplification. Polymerase chain reaction amplification is preferred.

In one embodiment of the present invention the NS5A region of the genome of at least one classical swine fever virus (CSFV) or fragments thereof is amplified prior to detection.

In one embodiment of the amplification product at least comprises a sequence which has a sequence identity of at least 70% to the region of nucleotides 9447 to 9462 according to SEQ ID NO. 1, preferably the region has a sequence identity of more than 80%, more preferably more than 90%, even more preferably more than 95% (including but not limited to more than 96%, more than 97%, more than 98%, more than 99%, more than 99.5% and 100% sequence identity). In a preferred embodiment the amplified region at least comprises the sequence according to SEQ ID NO. 2.

In a preferred embodiment the amplification product has at least 70% sequence identity to the nucleotides 8423 to 9913 according to SEQ ID NO. 1, preferably said region has at least 80% sequence identity to the nucleotides 9400 to 9505 according to SEQ ID NO. 1, more preferably at least 90% sequence identity, even more preferably at least 95% sequence identity (including but not limited to at least 96% sequence identity, at least 97% sequence identity, at least 98% sequence identity, at least 99% sequence identity, at least 99.5% sequence identity and 100% sequence identity).

In a further embodiment the amplification product has a sequence identity of at least 70% nucleotides 9388 to 9507 according to SEQ ID NO. 1, preferably at least 80% sequence identity to the nucleotides 9400 to 9505 according to SEQ ID NO. 1, more preferably at least 90% sequence identity, even more preferably at least 95% sequence identity (including but not limited to at least 96% sequence identity, at least 97% sequence identity, at least 98% sequence identity, at least 99% sequence identity, at least 99.5% sequence identity and 100% sequence identity).

In a further preferred embodiment the amplification product has a sequence identity of at least 70% to the nucleotides 9400 to 9505 according to SEQ ID NO. 1, preferably said region has at least 80% sequence identity to the nucleotides 9400 to 9505 according to SEQ ID NO. 1, more preferably at least 90% sequence identity, even more preferably at least 95% sequence identity (including but not limited to at least 96% sequence identity, at least 97% sequence identity, at least 98% sequence identity, at least 99% sequence identity, at least 99.5% sequence identity and 100% sequence identity).

In a further preferred embodiment at least one of the regions of the regions selected from the group of sequences consisting of SEQ ID NO.s 8, 9, 10, 11, 12, 13, 14, 15, 16, 17, 18, 19, 20, 21, 22, 23, 24, 25, 26, 27, 28 ,29, 30, 31, 32, 33, 34, 35, 36, 37, 38, 39, 40, and 41 is amplified prior to detection. In a further embodiment at least one amplified region comprises at least one sequence selected from group of sequences of nucleotides 13 to 118 of any one of the sequences according to SEQ ID NOs 8, 9, 10, 11, 12, 13, 14, 15, 16, 17, 18, 19, 20, 21, 22, 23, 24, 25, 26, 27, 28 ,29, 30, 31, 32, 33, 34, 35, 36, 37, 38, 39, or 40, or nucleotides 1 to 107of SEQ ID 41.

In one embodiment the amplification product has a length of 20 to 1500 nucleotides, preferably 40 to 1000 nucleotides, more preferably 50 to 500 nucleotides, even more preferably 80 to 150 nucleotides, most preferred the amplification product has a length of 90 to 120 nucleotides.

The person skilled in the art knows how to design and manufacture primers suited for the amplification as outlined above, i.e. oligonucleotide primers. Oligonucleotide primers may be prepared using any suitable method, such as, for example, the phosphotriester and phosphodiester methods or automated embodiments thereof. In one such automated embodiment diethylophosphoramidites are used as starting materials and may be synthesized as described by Beaucage et al., Tetrahedron Letters, 22:1859-1862 (1981). One method for synthesizing oligonucleotides on a modified solid support is described in U.S. Pat. No. 4,458,006. It is also possible to use a primer which has been isolated from a biological source (such as a restriction endonuclease digest).

In a further embodiment the at least one CSFV amplification primer is suited for amplifying a region of a classical swine fewer virus genome (CSFV), wherein the amplification product has a sequence identity of at least 70% to any one of the sequences selected from the group consisting of nucleotides 8423 to 9913 according to SEQ ID NO. 1, nucleotides 9388 to 9507 according to SEQ ID NO. 1, nucleotides 9400 to 9505 according to SEQ ID NO. 1, and nucleotides 9447 to 9462 according to SEQ ID NO. 1. Preferably said nucleic acid has a sequence identity of at least 80% sequence identity to any one of the sequences selected from the group consisting of nucleotides 8423 to 9913 according to SEQ ID NO. 1, nucleotides 9388 to 9507 according to SEQ ID NO. 1, nucleotides 9400 to 9505 according to SEQ ID NO. 1, and nucleotides 9447 to 9462 according to SEQ ID NO. 1, more preferably at least 90% sequence identity, even more preferably at least 95% sequence identity (including but not limited to at least 96%, at least 97%, at least 98%, at least 99%, at least 99.5% and 100% sequence identity).

Preferred amplification primers have a length of from about 15-100, more preferably about 20-50, most preferably about 20-40 bases. He is also aware of the possibility to design degenerated primer in order to allow hybridization of the primer to a variety of sequences differing in sequence at one or more positions. The skilled artisan easily recognizes that any primers that are suited for the amplification of fragments of the genome of a classical swine fewer virus (CSFV) as outlined above can be used in the method according to the present invention.

In a preferred embodiment the primers for amplification (CSFV amplification primer) comprise a 3'end consisting of at least the last 6 nucleotides according to the sequence of SEQ ID NO. 3 or SEQ ID NO. 4, respectively, preferably of at least the last 8 nucleotides, more preferably of at least the last 10 nucleotides. In further preferred embodiment the 3'ends of the at least two CSFV amplification primer consist of the sequence according to the sequence of SEQ ID NO. 3 or SEQ ID NO. 4, respectively. In yet another preferred embodiment the at least two CSFV amplification primer have the sequence according to SEQ ID NO. 3 or SEQ ID NO. 4, respectively. However, in one non limiting embodiment of the present invention amplification is performed using amplification primers having the sequence of SEQ ID NO. 3 and SEQ ID NO. 4.

In some cases it might be necessary to not only determine the presence of a classical swine fewer virus (CSFV) but also verify the isolation of the nucleic acid from the sample or to determine the relative amount classical swine fewer virus (CSFV) in a subject or a sample of a subject, respectively. For routine diagnostic the integration of controls verifying the RNA isolation step as well as the RT-PCR are of utmost importance. In 2005, a multiplex real-time RT-PCR assay with a heterologous internal control was described (Hoffmann, B., Depner, K., Schirrmeier, H., Beer, M., 2006. A universal heterologous internal control system for duplex real-time RT-PCR assays used in a detection system for pestiviruses. J. Virol. Methods 136 (1-2), 200-209). However, for analysing the status of the sample the amplification of nucleic acids from internal controls, e.g. house-keeping genes is preferred. Therefore, reference nucleic acids are included in a preferred embodiment of the present invention. Such a reference nucleic acid may be added externally or nucleic acids contained in the sample of the subject may serve as a reference nucleic acid. In latter case nucleic acids of so called housekeeping genes are preferred as they are most likely are abundant throughout all samples and are expressed substantially constant and are, therefore, well suited as reference nucleic acids. As the genome of classical swine fewer viruses (CSFV) consists of RNA, reference nucleic acids preferably consist of RNA, e.g. mRNA of said housekeeping genes. Nevertheless, the reference nucleic acid may also consist of DNA or any other nucleic acid as outlined above. In a further embodiment the reference nucleic acid consists of RNA and is reverse transcribed to cDNA. In a preferred embodiment the reference nucleic acid is an mRNA of one or more genes selected from the group consisting of β-actin, Glyceraldehyd-3-phosphate dehydrogenase (GAPDH), 18S rRNA, ß 2 microglobulin. In a further preferred embodiment of the present invention said reference nucleic acid is the mRNA of β-actin (SEQ ID NO. 42).

Reference nucleic acid probes and/or reference amplification primers might be necessary for the detection of the reference nucleic acid. The skilled artisan knows how to design suited primers and/or probes. Different regions of the reference nucleic acid may serve for the detection and the respective reference amplification primers and/or reference nucleic acid probes can be designed by those skilled in the art. In a preferred embodiment the mRNA of β-actin is determined by using the reference amplification primers, said reference amplification primers comprising 3'-ends consisting of at least the last 6 nucleotides of the sequence according to SEQ ID NO. 5 and SEQ ID NO. 6, respectively. In preferred embodiment the reference amplification primers have 3'-ends consisting of the sequences according to SEQ ID NO. 5 and SEQ ID NO. 6, respectively. In one embodiment the reference nucleic acid probe comprises the sequence according to SEQ ID NO. 7 and is between 5 and 40 nucleotides in length. In a preferred embodiment the reference nucleic acid probe has the sequence according to SEQ ID NO. 7.

In a further embodiment the nucleic acid probe and/or one or more CSFV amplification primer and/or reference nucleic acid probes and/or reference amplification primers may by labelled with fluorescent dyes. In the context of the present invention, fluorescent dyes may for example be FAM (5-or 6-carboxyfluorescein), VIC, NED, fluorescein, fluorescein isothiocyanate (FITC), IRD-700/800, cyanine dyes, also as CY3, CY5, CY3.5, CY5.5, Cy7, xanthen, 6-carboxy-2',4',7',4,7-hexachlorofluorescein (HEX), TET, 6-carboxy-4',5'-dichloro-2',7'-dimethodyfluorescein (JOE), N,N,N',N'-Tetramethyl-6-carboxyrhodamine (TAMRA), 6-carboxy-X-rhodamine (ROX), 5-Carboxyrhodamine-6G (R6G5), 6-carboxyrhodamine-6G (RG6), Rhodamine, Rhodamine Green, Rhodamine Red, Rhodamine 110, BODIPY dyes, such as BODIPY TMR, Oregon Green, coumarines such as Umbelliferone, benzimides, such as Hoechst 33258; phenanthridines, such as Texas Red, Yakima Yellow, Alexa Fluor, PET, ethidium bromide, acridinium dyes, carbazol dyes, phenoxazine dyes, porphyrine dyes, polymethin dyes, and the like.

Different methods for the amplification and/or detection of nucleic acids are known to those skilled in the art. Any method may be applied by which nucleic acids may be detected specifically, i.e. detection of specific sequences within said nucleic acid. In a preferred embodiment of the present invention the detection and/or amplification of the NS5A region and/or the reference nucleic acid is performed by one or more of the methods selected from a group consisting of sequencing (including Sanger method, shot-gun sequencing and pyro sequencing *in-situ* hybridisation, Northern blot, polymerase chain reaction (PCR), quantitative PCR (qPCR), real-time PCR, reverse transcriptase PCR (RT-PCR), real-time RT-PCR, Microarray analysis.

The real-time PCR is a method to simultaneously amplify and quantify nucleic acids using a polymerases chain reaction (PCR). Real-time reverse transcription PCR (RT-PCR) is a real-time PCR method further comprising a reverse transcription of RNA into DNA, e.g. mRNA into cDNA. In qPCR methods, the amplified nucleic acid is quantified as it accumulates. Typically, fluorescent dyes that intercalate with double-stranded DNA (e.g. ethidiumbromide or SYBR® Green I) or labelled nucleic acid probes ("reporter probes") that fluoresce when hybridized with a complementary nucleic acid (e.g. the accumulating DNA) are used for detection and/or quantification in PCR based methods. Particularly, fluorogenic primers, hybridization probes (e.g. LightCycler probes (Roche)), hydrolysis probes (e.g. TaqMan probes (Roche)), or hairpin probes, such as molecular beacons, Scorpion primers (DxS), Sunrise primers (Oncor), LUX primers (Invitrogen), Amplifluor primers (Intergen) or the like can be used as reporter probes. In accordance with the present invention, fluorogenic primers or probes may for example be primers or probes to which fluorescence dyes have been attached, e.g. covalently attached. Such fluorescence dyes may for example be FAM (5-or 6-carboxyfluorescein), VIC, NED, Fluorescein, FITC, IRD-700/800, CY3, CY5, CY3.5, CY5.5, HEX, TET, TAMRA, JOE, ROX, BODIPY TMR, Oregon Green, Rhodamine Green, Rhodamine Red, Texas Red, Yakima Yellow, Alexa Fluor, PET Biosearch Blue™, Marina Blue®, Bothell Blue®, CAL Fluor® Gold, CAL Fluor® Red 610, Quasar™ 670, LightCycler Red640®, Quasar™ 705, LightCycler Red705® and the like. Particular reporter probes may additionally comprise fluorescence quenchers.

In particular embodiments of the invention the polymerase used for PCR based methods is a polymerase from a thermophile organism or a thermostable polymerase or is selected from the group consisting of *Thermus thermophilus* (Tth) DNA polymerase, *Thermus acquaticus* (Taq) DNA polymerase, *Thermotoga maritima* (Tma) DNA polymerase, *Thermococcus litoralis* (Tli) DNA polymerases, *Pyrococcus furiosus* (Pfu) DNA polymerase, *Pyrococcus woesei* (Pwo) DNA polymerase, *Pyrococcus kodakaraensis* KOD DNA polymerase, *Thermus filiformis* (Tfi) DNA polymerase, *Sulfolobus solfataricus* Dpo4 DNA polymerase, *Thermus pacificus* (Tpac) DNA polymerase, *Thermus eggertssonii* (Teg) DNA polymerase, *Thermus brockianus* (Tbr) and *Thermus flavus* (Tfl) DNA polymerase.

The host for the classical swine fewer virus (CSFV) are swine. However, it might be necessary to determine the presence of the virus in any other species, e.g. humans which come into contact with swines. Thus, in one embodiment the subject according to the present invention is a mammal, e.g. a human, cattle, cat, dog, horse, or swine. In a preferred embodiment the subject is a swine.

The present invention further relates to a kit for determining the presence of a classical swine fewer virus (CSFV) in a subject comprising:
- a nucleic acid probe which is capable of hybridising to the NS5A region of a classical swine fewer virus (CSFV) or parts thereof and/or to a nucleic acid complementary to the NS5A region of classical swine fewer virus (CSFV) or parts thereof and/or at least one primer for sequencing of the NS5A region of the at least one classical swine fewer virus (CSFV) or parts thereof.

The kit may optionally further comprises at least one primer for the amplification of the genome of a classical swine fewer virus (CSFV amplification primer). In a preferred embodiment the kit comprises at least one CSFV amplification primer for the amplification of the genome of a CSFV or parts thereof. The kit additionally and optionally may comprise reference nucleic acid probes and reference amplification primers according to the present invention

The nucleic acid probe, CSFV amplification primer, reference nucleic acid probes and reference amplification primers of the kit have the same embodiments and features as outlined herein above.

In one embodiment the kit is a kit for performing real-time RT-PCR. Thus, in this embodiment the kit according to the present invention further comprises reagents for conducting real-time RT-PCR. Such reagents include buffers, dNTPs, and enzymes (such as polymerases) for performing real-time RT-PCR. The skilled artisan is aware of reagents needed for performing real-time RT-PCR.

The term "polymerase" refers to an enzyme that synthesizes nucleic acid strands (e.g., RNA or DNA) from ribonucleoside triphosphates or deoxynucleoside triphosphates.

A variety of polypeptides having polymerase activity are useful in accordance with the present invention. Included among these polypeptides are enzymes such as nucleic acid polymerases (including DNA polymerases and RNA polymerases). Such polymerases include, but are not limited to, *Thermus thermophilus* (Tth) DNA polymerase, *Thermus aquaticus* (Taq) DNA polymerase, *Thermotoga neopolitana* (Tne) DNA polymerase, *Thermotoga maritima* (Tma) DNA polymerase, *Thermococcus litoralis* (Tli or VENT™.) DNA polymerase, *Pyrococcus furiosus* (Pfu) DNA polymerase, DEEPVENT™ DNA polymerase, *Pyrococcus woosii* (Pwo) DNA polymerase, *Pyrococcus sp* KDD2 (KOD) DNA polymerase, *Bacillus sterothermophilus* (Bst) DNA polymerase, *Bacillus caldophilus* (Bca) DNA polymerase, *Sulfolobus acidocaldarius* (Sac) DNA polymerase, *Thermus eggertssonii* (Teg), *Thermoplasma acidophilum* (Tac) DNA polymerase, *Thermus flavus* (Tfl/Tub) DNA polymerase, *Thermus ruber* (Tru) DNA polymerase, *Thermus brockianus* (DYNAZYME) DNA polymerase, *Methanobacterium thermoautotraphicum* (Mth) DNA polymerase, mycobacterium DNA polymerase (Mtb, Mlep), and mutants, variants and derivatives thereof. RNA polymerases such as T3, T5 and SP6 and mutants, variants and derivatives thereof may also be used in accordance with the invention.

The nucleic acid polymerases used in the present invention may be mesophilic or thermophilic, and are preferably thermophilic. Preferred mesophilic DNA polymerases include T7 DNA polymerases, T5 DNA polymerase, Klenow fragment DNA polymerase, DNA polymerase III and the like. Preferred thermostable DNA polymerases that may be used in the methods and kits of the invention include Teg, Taq, Tne, Tma, Pfu, Tfl, Tth, Stoffel fragment, VENT™ and DEEPVENT™ DNA polymerases, and mutants, variants and derivatives thereof (U.S. Pat. No. 5,436,149; U.S. Pat. No. 4,889,818; U.S. Pat. No. 4,965,188; U.S. Pat. No. 5,079,352; U.S. Pat. No. 5,614,365; U.S. Pat. No. 5,374,553; U.S. Pat. No. 5,270,179; U.S. Pat. No. 5,047,342; U.S. Pat. No. 5,512,462; WO 92/06188; WO 92/06200; WO 96/10640; Barnes, W. M., Gene 112:29-35 (1992); Lawyer, F. C., et al., PCR Meth. Appl. 2:275-287 (1993); Flaman, J.-M, et al., Nucl. Acids Res. 22(15):3259-3260 (1994)). Examples of DNA polymerase substantially lacking in 3' exonuclease activity include, but are not limited to, Taq, Tne^{exo-}, Tma^{exo-}, Pfu^{exo-}, Pwo^{exo-} and Tth DNA polymerases, and mutants, variants and derivatives thereof.

Polypeptides having reverse transcriptase activity for use in the invention include any polypeptide having reverse transcriptase activity. Such enzymes include, but are not limited to, retroviral reverse transcriptase, retrotransposon reverse transcriptase, hepatitis B reverse transcriptase, cauliflower mosaic virus reverse transcriptase, bacterial reverse transcriptase, Tth DNA polymerase, Taq DNA polymerase (Saiki, R. K., et al., Science 239:487-491 (1988); U.S. Pat. Nos. 4,889,818 and 4,965,188), Tne DNA polymerase (WO 96/10640), Tma DNA polymerase (U.S. Pat. No. 5,374,553) and mutants, variants or derivatives thereof (see, e.g., copending U.S. patent application Ser. Nos. 08/706,702 and 08/706,706, of A. John Hughes and Deb K. Chattedee, both filed Sep. 9, 1996, which are incorporated by reference herein in their entireties). Preferred enzymes for use in the invention include those that are reduced or substantially reduced in RNase H activity. By an enzyme "substantially reduced in RNase H activity" is meant that the enzyme has less than about 20%, more preferably less than about 15%, 10% or 5%, and most preferably less than about 2%, of the RNase H activity of the corresponding wildtype or RNase H⁺ enzyme such as wildtype Moloney Murine Leukemia Virus (M-MLV), Avian Myeloblastosis Virus (AMV) or Rous Sarcoma Virus (RSV) reverse transcriptases. The RNase H activity of any enzyme may be determined by a variety of assays, such as those described, for example, in U.S. Pat. No. 5,244,797, in Kotewicz, M. L., et al., Nucl. Acids Res. 16:265 (1988) and in Gerard, G. F., et al., FOCUS 14(5):91 (1992), the disclosures of all of which are fully incorporated herein by reference. Particularly preferred such polypeptides for use in the invention include, but are not limited to, M-MLV H reverse transcriptase, RSV H⁻ reverse transcriptase, AMV H⁻ reverse transcriptase, RAV (Rous-associated virus) H⁻ reverse transcriptase, MAV (myeloblastosis-associated virus) H- reverse transcriptase and HIV H⁻ reverse transcriptase. It will be understood by one of ordinary skill, however, that any enzyme capable of producing a DNA molecule from a ribonucleic acid molecule (i.e., having reverse transcriptase activity) that is substantially reduced in RNase H activity may be equivalently used in the compositions, methods and kits of the invention.

DNA and RNA polymerases for use in the invention may be obtained commercially, for example from QIAGEN (Hilden, Germany), Life Technologies, Inc. (Rockville, Md.), New England BioLabs (Beverly, Mass.) or ROCHE Biochemicals. Polypeptides having reverse transcriptase activity for use in the invention may be obtained commercially, for example from QIAGEN (Hilden, Germany), Life Technologies, Inc. (Rockville, Md.), Pharmacia (Piscataway, N.J.), Sigma (Saint Louis, Mo.) or ROCHE (Penzberg, Germany). Alternatively, polypeptides having reverse transcriptase activity may be isolated from their natural viral or bacterial sources according to standard procedures for isolating and purifying natural proteins that are well-known to one of ordinary skill in the art (see, e.g., Houts, G. E., et al., J. Virol. 29:517 (1979)). In addition, the polypeptides having reverse transcriptase activity may be prepared by recombinant DNA techniques that are familiar to one of ordinary skill in the art (see, e.g., Kotewicz, M. L., et al., Nucl. Acids Res. 16:265 (1988); Soltis, D. A., and Skalka, A. M., Proc. Natl. Acad. Sci. USA 85:3372-3376 (1988)).

As used herein, the term "dNTP" refers to desoxyribonucleoside triphosphates. Non-limiting examples of such dNTPs are dATP, dGTP, dCTP, dTTP, dUTP, which may also be present in the form of labelled derivatives, for instance comprising a fluorescence label, a radioactive label, a biotin label. dNTPs with modified nucleotide bases are also encompassed, wherein the nucleotide bases are for example hypoxanthine, xanthine, 7-methylguanine, inosine, xanthinosine, 7-methylguanosine, 5,6-dihydrouracil, 5-methylcytosine, pseudouridine, dihydrouridine, 5-methylcytidine. Furthermore, ddNTPS of the above-described molecules are encompassed in the present invention.

The kit according to the present invention in one embodiment includes a positive control nucleic acid suited to prove the efficiency of the CSFV amplification primers and nucleic acid probes. In one embodiment said positive control nucleic acid comprises a first primer binding sequence, a second primer binding sequence and a probe binding sequence, wherein the first and the second primer binding sequence are selected and oriented so that the sequence located in-between can be amplified by the at least two CSFV amplification primers; wherein the probe binding sequence comprises a part of the NS5A region of a classical swine fewer virus (CSFV) to which said nucleic acid probe hybridizes; and wherein the probe binding sequence is located in-between the first and second primer binding sequence comprises. In a preferred embodiment of the present invention the positive control nucleic acid is RNA or DNA.

In a further preferred embodiment the positive control nucleic acid contained in the kit according to the present invention comprises a transcription initiation sequence. In context of the present invention a "transcription initiation sequence" is a sequence which promotes the (reverse)-transcription from RNA to DNA or DNA to RNA by an enzyme, respectively. In one embodiment of the present invention the positive control nucleic acid contained in the kit according to the present invention consists of DNA and is transcribed to RNA. The transcribed RNA is then used as a positive control in the real-time RT-PCR reaction. Thus, in a preferred embodiment the positive control nucleic acid contained in the kit according to the present invention consists of DNA and comprises a transcription initiation sequence promoting the transcription from DNA to RNA. Transcription initiation sequences are well known by those skilled in the art. Gene promoters are examples of such transcription initiation sequences. Gene promoters contain specific DNA sequences and/or response elements which provide a binding site for RNA polymerase and/or transcription factors that recruit RNA polymerase. In a preferred embodiment the transcription initiation sequence is selected from the group consisting of T7 promoter, T3 promoter and SP6 promoter. The transcription initiation sequence is positioned and arranged within the positive control nucleic acid in a way so after transcription or reverse transcription with an enzyme a transcribed or reveres transcribed positive control nucleic acid may be obtained which comprises said first primer binding sequence, said second primer binding sequence and said probe binding sequence, wherein the first and the second primer binding sequence are selected and oriented so that the sequence located in-between can be amplified by the at least two CSFV amplification primers; wherein the probe binding sequence comprises a part of the NS5A region of a classical swine fewer virus (CSFV) to which said nucleic acid probe hybridizes; and wherein the probe binding sequence is located in-between the first and second sequence comprises the probe binding sequence.

In a preferred embodiment of the present invention the positive control nucleic acid comprises the sequence of SEQ ID NO. 43. In a further embodiment the positive control nucleic acid has the sequence of SEQ ID NO. 43

The present invention further relates to an isolated nucleic acid comprising a 3'end consisting of at least the last 6 nucleotides according to the sequence of SEQ ID NO. 3 or SEQ ID NO. 4, respectively, preferably of at least the last 8 nucleotides, more preferably of at least the last 10 nucleotides. In further preferred embodiment the 3'ends of the nucleic acid consist of the sequence according to the sequence of SEQ ID NO. 3 or SEQ ID NO. 4.

The present invention also relates to an isolated nucleic acid of 16 to 40 nucleotides in lengths, wherein the nucleic acid comprises the sequence according to SEQ ID NO. 2. In a preferred embodiment the nucleic acid has the sequence according to SEQ ID NO. 2. In a preferred embodiment the isolated nucleic acid has the embodiments of the nucleic acid probe for the detection of CSFV as outlined herein above.

The present invention further relates to a nucleic acid comprising the sequence according to SEQ ID NO. 43. Preferably the nucleic acid has the sequence according to SEQ ID NO. 43.

As outlined above the inventors where able to identify new, unknown classical swine fewer virus (CSFV) strains by using the method according to the present invention. Preferred regions of said new, unknown classical swine fewer viruses (CSFV) are given in SEQ ID NO.s 37 to 41. Thus, the present invention further relates to an isolated nucleic acid comprising a sequence according to any one of the sequences selected from the group consisting of SEQ ID NO. 37, 38, 39, 40 and 41.

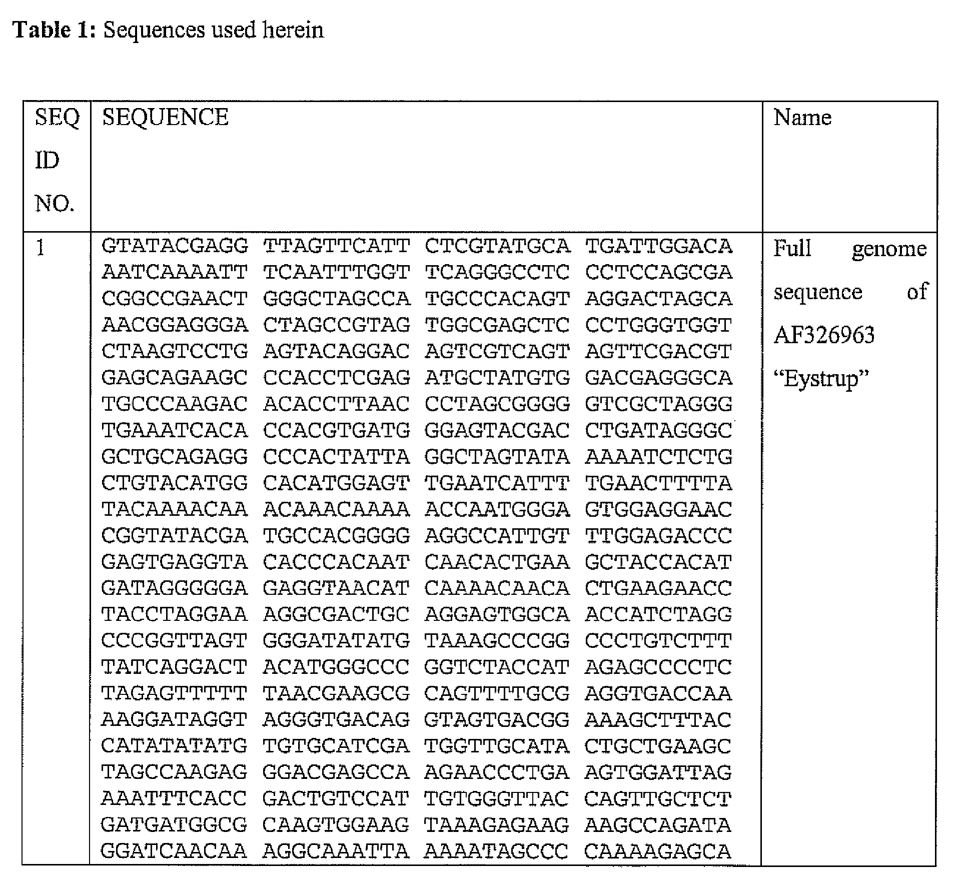

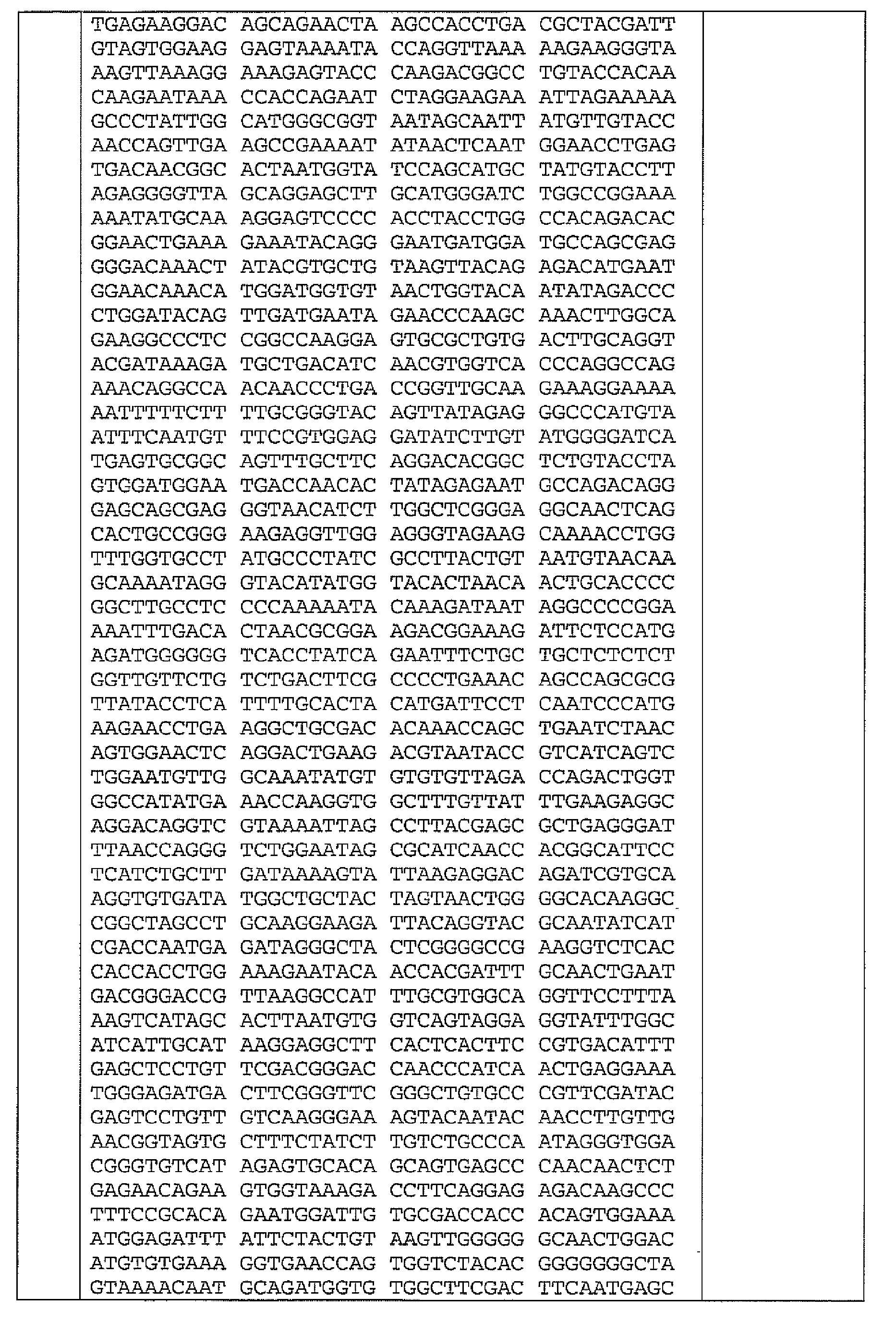

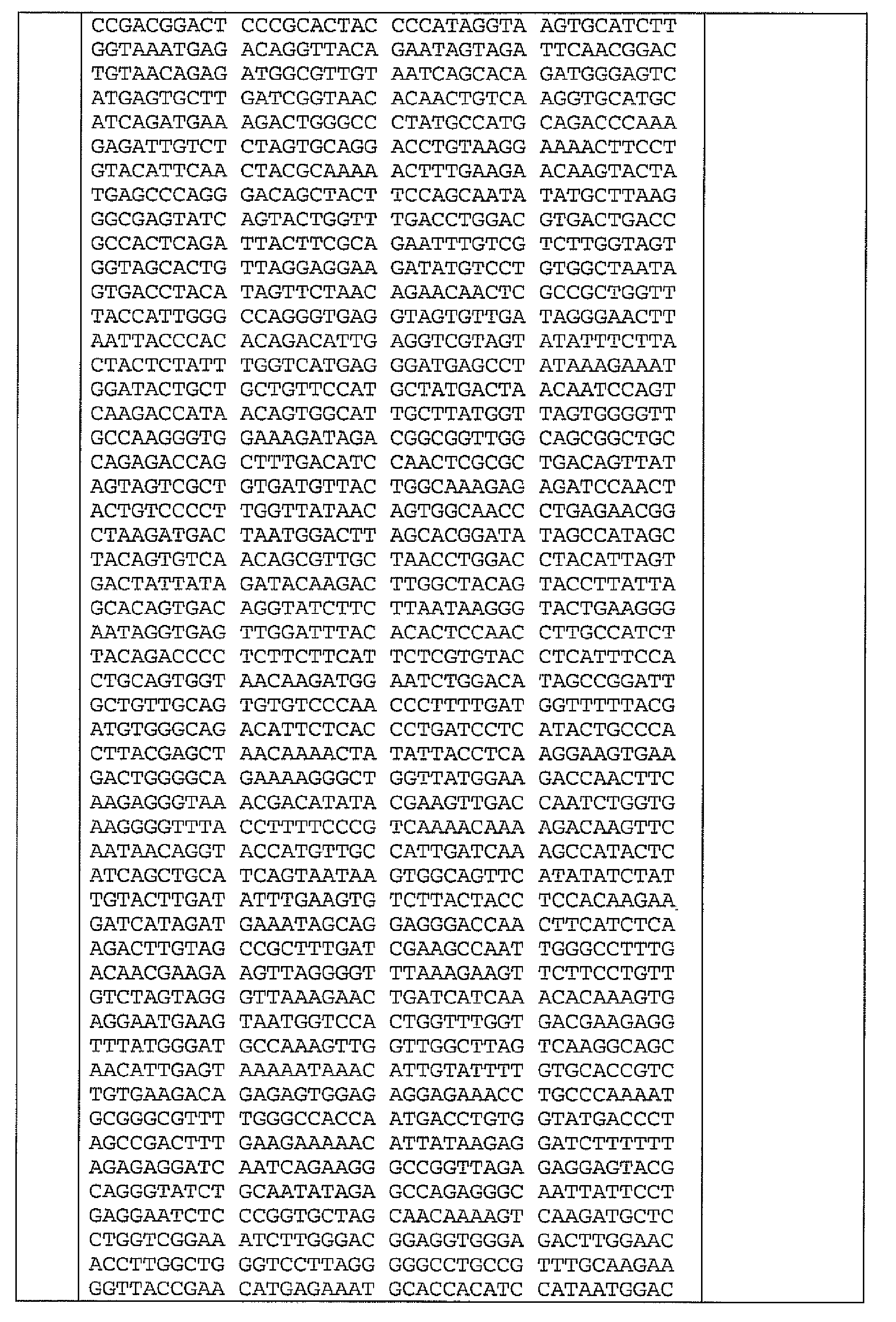

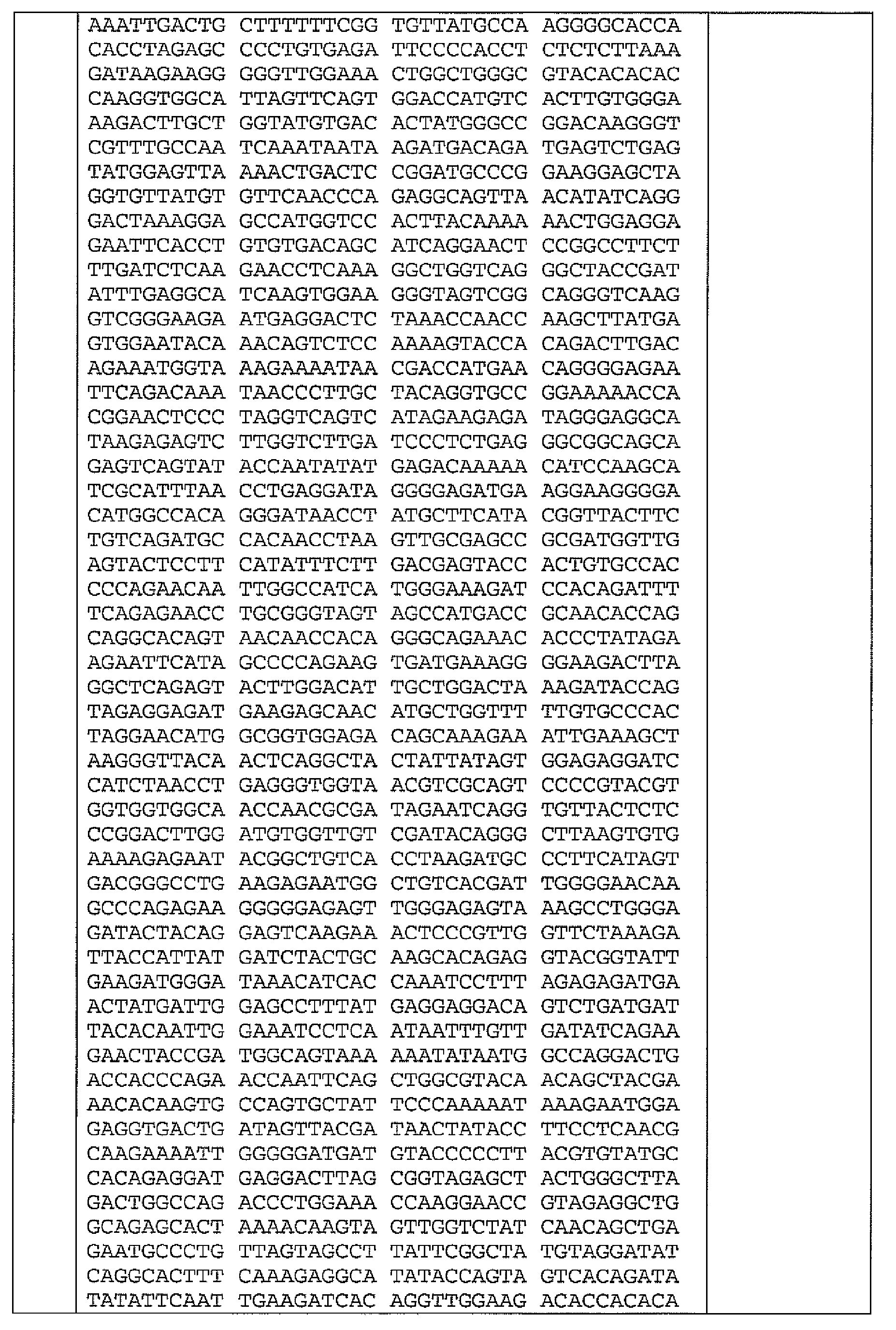

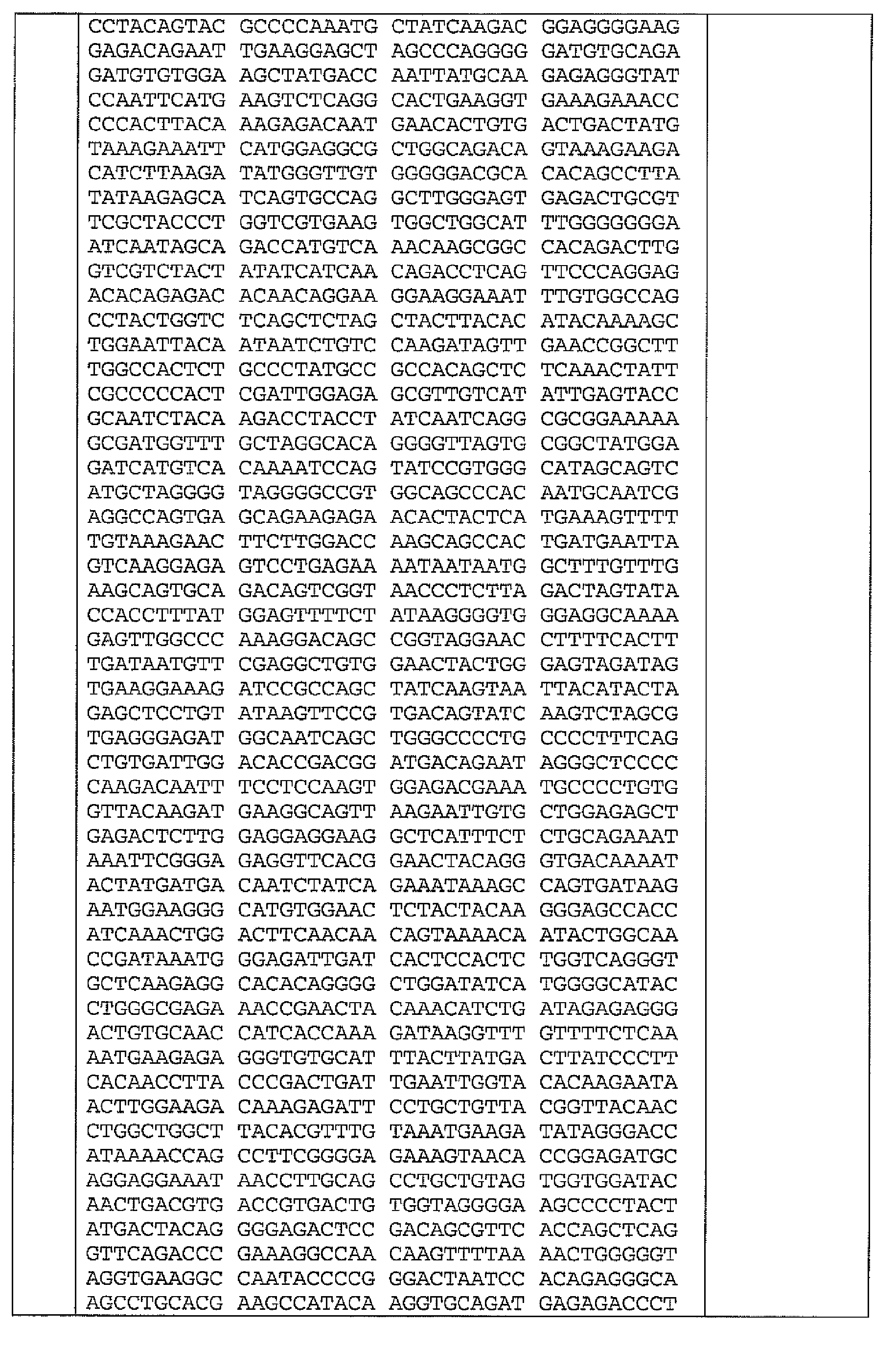

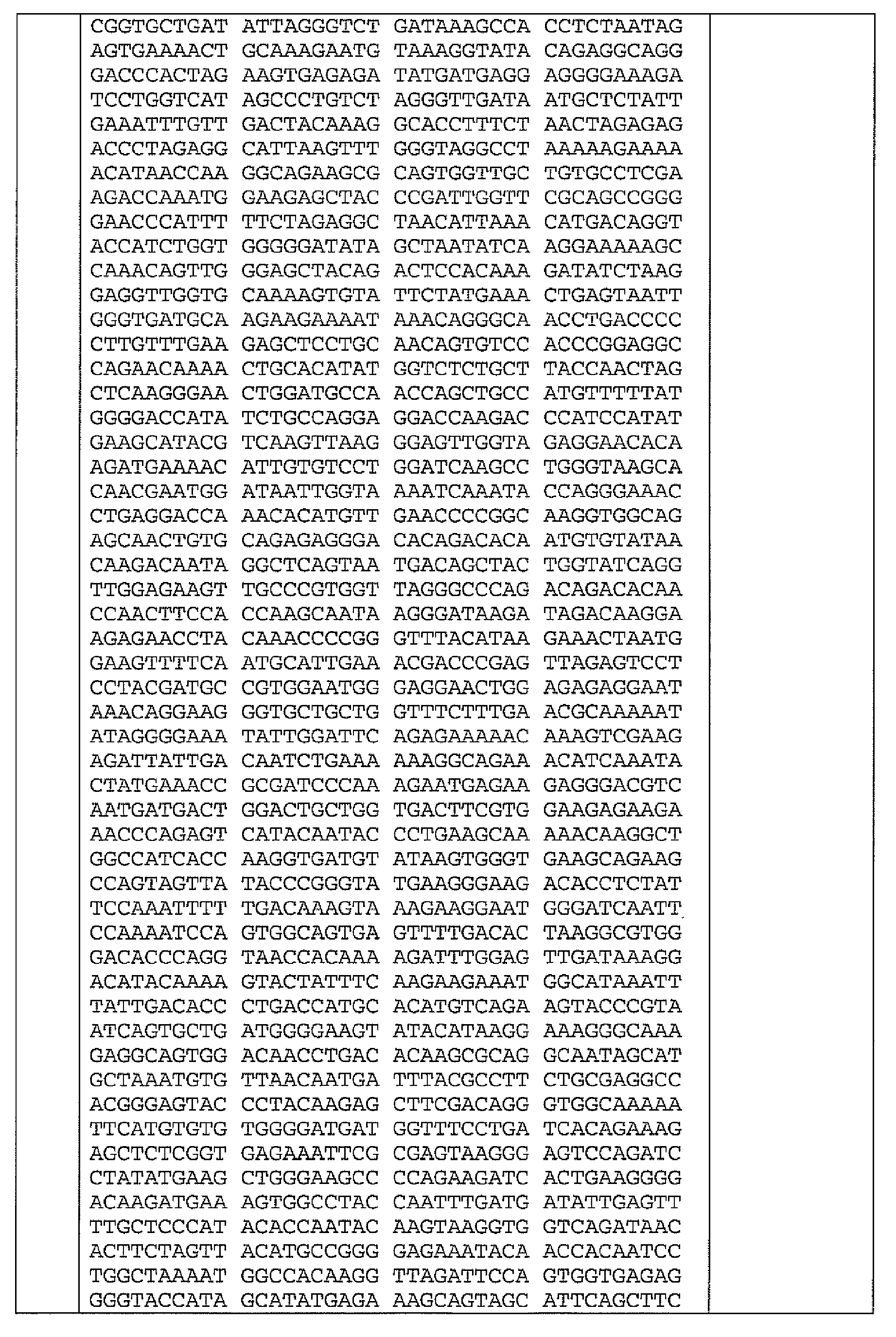

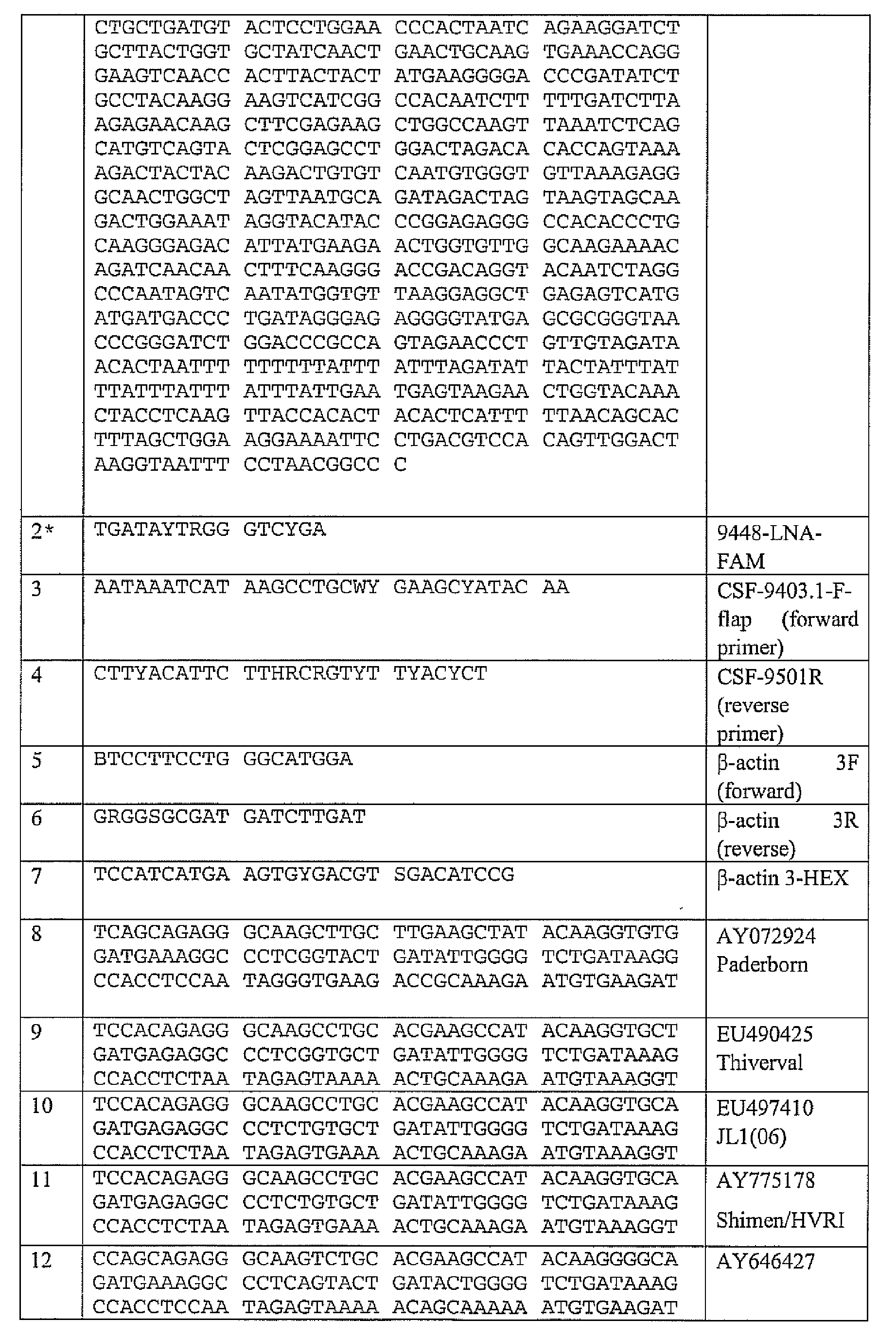

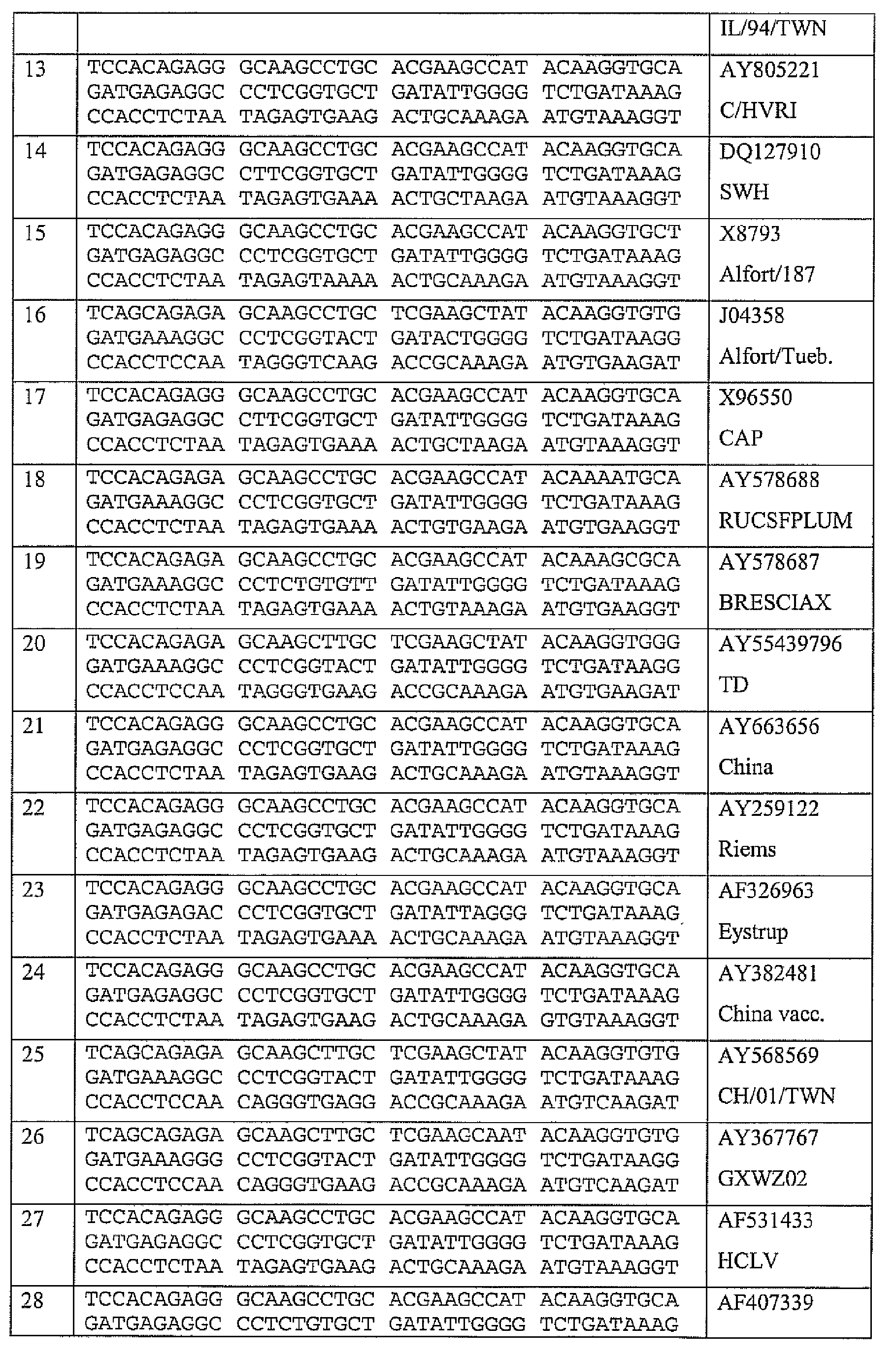

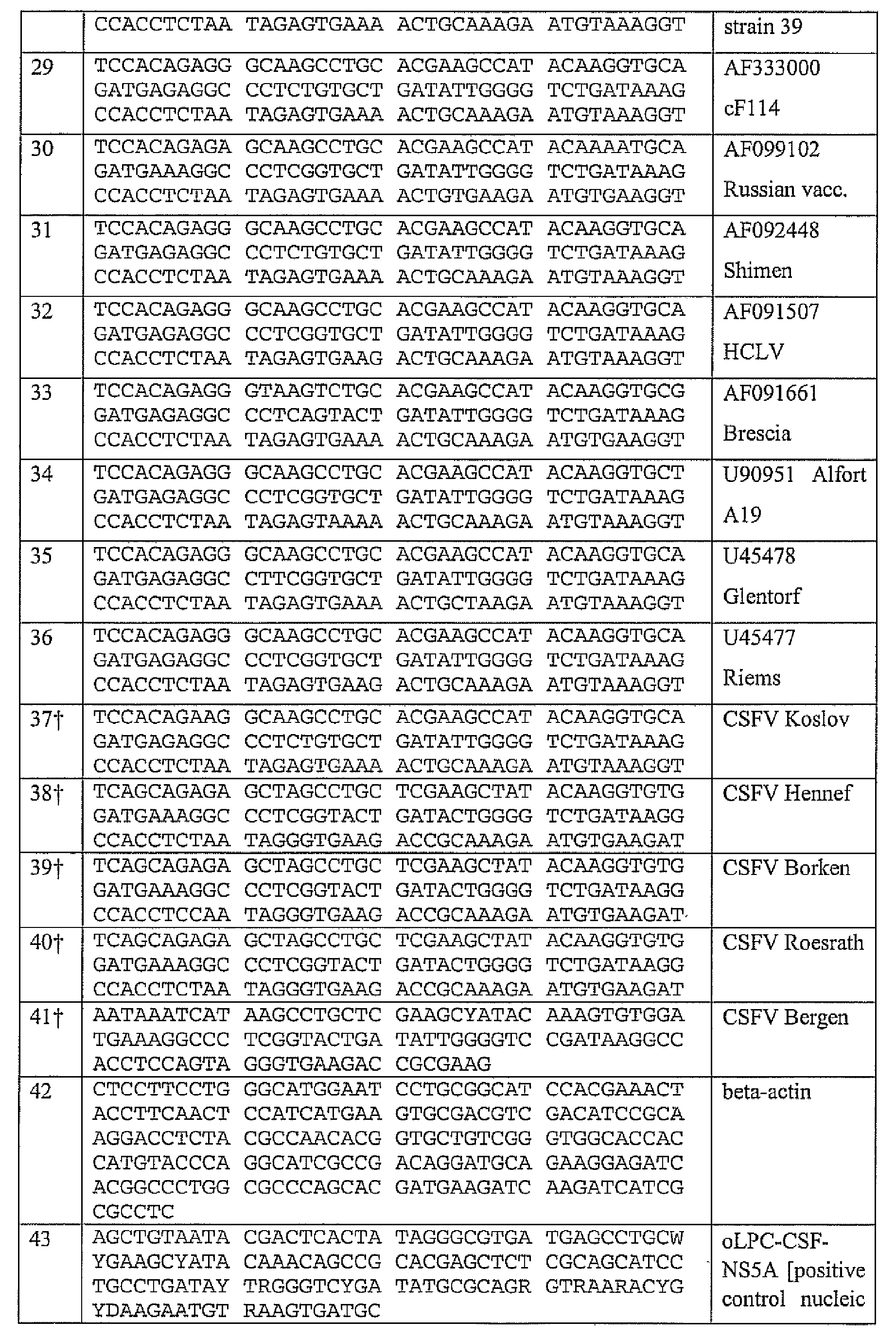

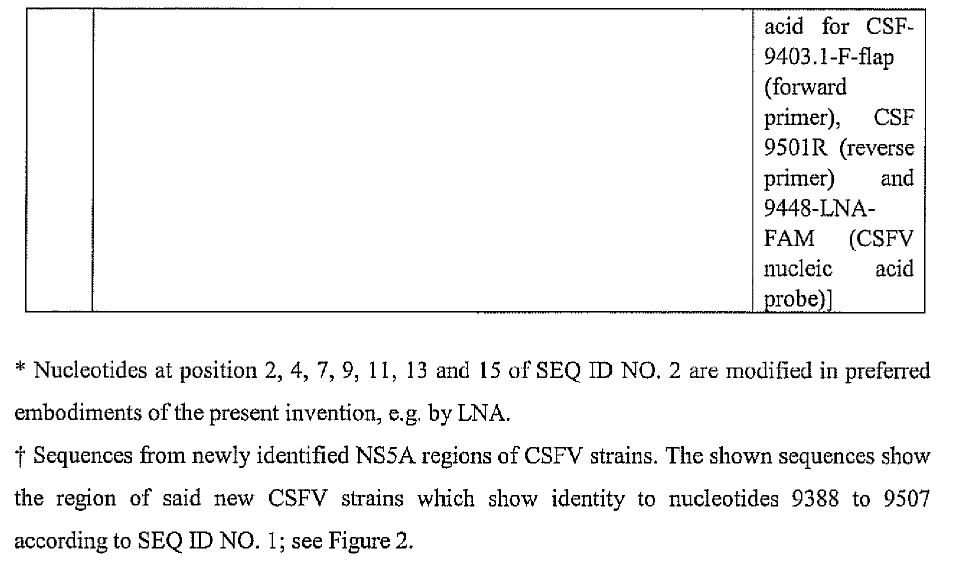

### Examples

### Samples

BDV, BVDV and CSFV strains were obtained from the respective German national reference laboratories. CSFV strains were cultured using porcine kidney cells (PK15), BVDV and BDV were propagated on bovine kidney cells (MDBK) or sheep thymus cells (SFT), respectively, according to the standard protocols (Collection of Cell Lines in Veterinary Medicine, Friedrich-Loeffler-Institut [FLI], Insel Rims, Germany). Recent positive and negative samples from wild boar in CSFV-positive regions in Western Germany submitted by state veterinary services were tested. The EPIZONE reference RNA panel developed at the FLI incorporates a representative collection of CSFV RNA samples of the most relevant genotypes as well as members of related pestiviruses (BDV, BVDV, and atypical pestiviruses). The panel currently comprises 31 different pestiviral RNA samples extracted from cell culture supernatants at a concentration of around 1,000 genome copies per µl. This panel was used to analyze the CSFV-specificity of the NS5A system.

### Development of a new NS5A standard

For determination of sensitivity, a new RNA standard was generated. A 140bp-long oligonucleotide (oLPC-CSF-NS5A) containing a T7 promoter and the primer and probe binding sequence of the NS5A specific real-time RT-PCR protocol connected with short spacer sequences served as template for in-vitro transcription of positive-sense single-strand RNA (Table 1). The in-vitro transcribed RNA product was treated with DNase and purified using the RNeasy® Mini Kit (Qiagen GmbH, Hilden, Germany). The concentration of RNA was determined, and a ten-fold dilution series was established.

### oLPC-CSF-NS5A:

agc tg TAA TAC GAC TCA CTA TAG GG cgtgatga
GCCTGCWYGAAGCYATACAA acag CCG CAC GAG CTC TCG CAG CAT
CC tgcc TGATAYTRGGGTCYGA tatgcgc AGR GTR AAR ACY GYD AAG
AAT GTR AAG tgatgc

### Partial sequencing of CSFV genomes

For sequencing, the viral RNA was amplified in a RT-PCR with the Super Script® III One-Step RT-PCR with Platinum® Taq Kit (Invitrogen, Carlsbad, CA, USA). DNA fragments were isolated from agarose gels with the QIAquick® Gel Extraction Kit (Qiagen). Sequencing was performed with the BigDye® Terminator v1.1 Cycle Sequencing Kit (Applied Biosystems, Foster City, CA, USA). Nucleotide sequences were read using the ABI 3130 Genetic Analyzer (Applied Biosystems) and for analysis the Genetic Computer Group software version 11.0 (Accelrys Inc., San Diego, CA, USA) was used.

### Real-time RT-PCR design

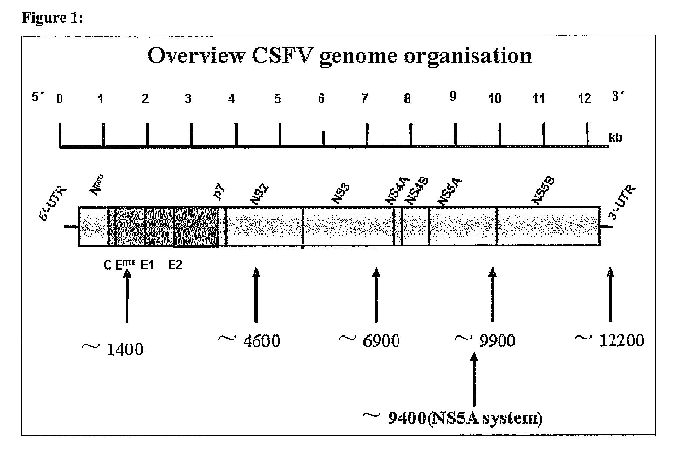

Different regions of the CSFV genome (E^{RNS}, NS2, NS3, NS5A and the 3'UTR) were tested for primer and probe selection (Figure 1). In order to obtain more sequence information to optimize sensitivity and specificity of the NS5A real-time RT-PCR system, the corresponding region of various CSF viruses of different genotypes was sequenced. These data together with the sequences in the NCBI database were used for designing new primers and probes. A number of primer combinations with different starting points, wobble bases or additional flap sequences as well as different probes (sense-, antisense- and locked nucleic acid [LNA] probes) were tested. The following primer combination turned out as optimal and is therefore a preferred embodiment of the present invention: CSF-9403.1-F-flap (forward primer containing an additional flap sequence and three wobble bases) and 9501R (reverse primer with seven wobble bases). The size of the amplified fragment with these primers is 98 bp. For detection, a FAM-labeled LNA probe with three wobble and seven LNA nucleotides is used (SEQ ID NO. 2; see Table 2 and Figure 2).

For the one-step real-time RT-PCR protocol the AgPath-ID™ One-Step RT-PCR Kit (Applied Biosystems) was used.

The 25 µl total reaction volume for the duplex real-time RT-PCR consists of: 12.5 µl 2x RT-PCR buffer, 2.5 µl RNase-free water, 1 µl 25x RT-PCR Enzyme mix, 2 µl β-actin mix 3-HEX (for detection of reference nucleic acid β-actin mRNA)) and 2 µl NS5A mix-FAM.

The CSFV-specific NS5A mix-FAM contains 12.5 pmol/µl forward primer, 10 pmol/µl reverse primer and 4 pmol/µl FAM-labeled probe. The mix for the internal control (β-actin mix 3) contains 2.5 pmol/µl each of forward and reverse primer and 1.25 pmol/µl HEX-labeled probe. Five µl of template RNA were added to 20 µl of master mix.

For the real-time RT-PCR the following thermal profile was used: 10 minutes at 45 °C (reverse transcription) and 10 minutes at 95°C (RT inactivation/polymerase activation) followed by 42 cycles of DNA amplification, each consisting of 15 seconds denaturation at 95 °C, 30 seconds annealing (with end-point fluorescence data collection) at 57 °C and 35 seconds elongation at 72 °C. To compare sensitivity and specificity both a CSFV-specific and a "panpesti" real-time RT-PCR system were used (Hoffmann et al., 2005; Hoffmann et al., 2006). Real-time RT-PCR was carried out using an Mx3005P cycler (Stratagene, La Jolla, CA, USA).

**Table 2: Primers and probes used in the CSFV-specific NS5A real-time RT-PCR system with β-actin mix 3 internal control. Locked nucleic acids (LNA) in probe sequences are marked by bold italics. The synthetic oligonucleotide used for generation of a NS5A RNA standard is listed as well.**

| **Primer name** | **Primer sequence** | **Additional informations** |
|---|---|---|
| CSF-9403.1-F-flap (forward primer) | AATAAATCATAAGCCTGCWYGAAGCYATAC AA | |
| CSF-9501R (reverse primer) | CTTYACATTCTTHRCRGTYTTYACYCT | NS5A specific system |
| 9448-LNA-FAM | TGATAYTRGGGTCYGA | |
| | | |
| CSF 100F (forward) | ATGCCCAYAGTAGGACTAGCA | |
| CSF 192R (reverse) | CTACTGACGACTGTCCTGTAC | Published by Hoffmann et al. 2005 |
| CSF 1-FAM | TGGCGAGCTCCCTGGGTGGTCTAAGT | |
| | | |
| ß-actin 3F (forward) | BTCCTTCCTGGGCATGGA | |
| ß-actin 3R (reverse) | GRGGSGCGATGATCTTGAT | Published by Moniwa et al. 2007 |
| ß-actin 3-HEX | TCCATCATGAAGTGYGACGTSGACATCCG | |

| | | |
|---|---|---|
| Bold letters refer to LNA-Nucleotides | | |

### Results of NS5A duplex real-time RT-PCR with internal control system

In order to test the CSFV-specificity of the new assay, the EPIZONE reference RNA panel was used, containing 13 CSFV RNA samples, and 17 BDV or BVDV RNA samples of all representative genotypes. Unlike the prior art CSF 1 system, all CSF viruses were clearly positive using the NS5A system (Table 3). All non-CSFV pestivirus RNAs did not deliver detectable amplification products with the NS5A system showing the specificity of the method according to the present invention. Furthermore, the NS5A system showed an improved sensitivity compared to the prior art system.

Current German field samples from recent CSFV outbreaks in wild boar were also tested with the new NS5A assay confirming in all cases the previously determined sample status (Hoffmann et al., 2005a) (data not shown). In addition, since recent CSF outbreaks in Germany and Europe were mostly associated with the CSFV genotype 2.3, RNA of 20 recent European CSFV isolates of that genotype were extracted and tested with the method according to the present invention (NS5A system). All samples scored clearly positive. Subsequently, these RNAs were also 1000-fold diluted and tested in the NS5A system and the prior art CSF 1 system, showing a reduction in Cq-value of about ten (Table 4).

In order to further test the sensitivity of the NS5A system, a RNA standard and two 10-fold dilutions series containing CSFV RNA of a) genotype 2.3 and b) genotype 1.1 were analyzed. In both dilution series, six steps scored positive with the NS5A assay, and the Cq-values were within 16 and 33 (genotype 1.1), respectively 20 and 38 (genotype 2.3). The in comparison tested prior art CSF1 system detected six steps of a C-strain "Riems" dilution series and five steps of the CSF 2.3 dilution series (data not shown). In both dilution series the NS5A assay was more sensitive than the CSF 1 system published by Hoffmann et al., 2005.

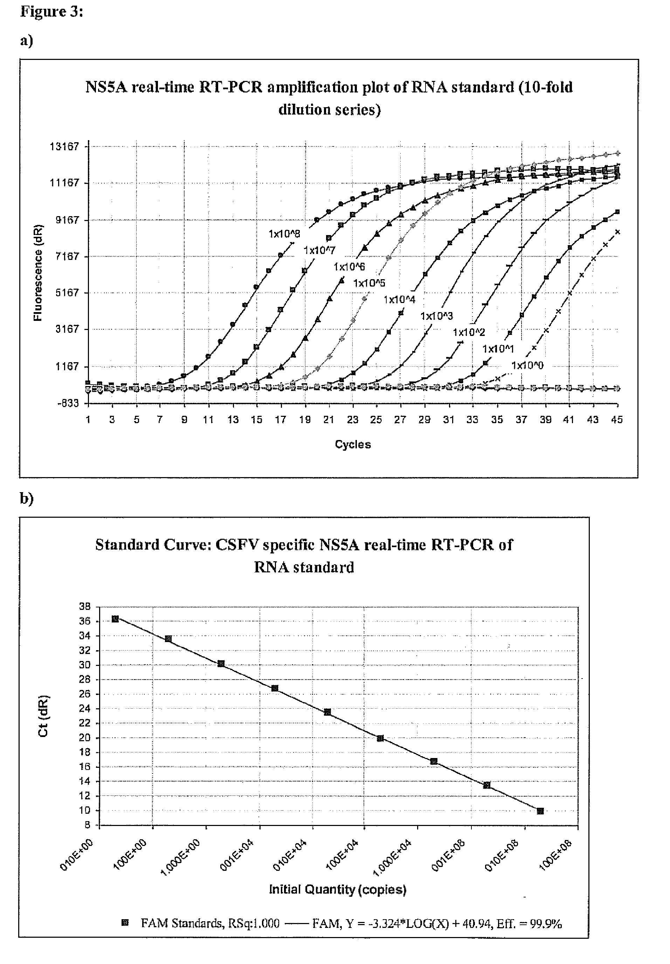

Furthermore, a dilution series of a new RNA standard, generated by *in vitro* transcription of a synthetic oligonucleotide was tested. Here, nine dilution steps from 108 to 100 copies were detected in the NS5A real-time RT-PCR (Figures 3a and 3b).

For routine diagnostics, the NS5A system was finally tested in a duplex real-time RT-PCR protocol with an internal control system using β-actin (mix 3). No remarkable influence concerning sensitivity or specificity in the duplex assays compared with a NS5A single assay was observed.

**Table 3: Real-time RT-PCR results in the NS5A and CSF1 systems for CSFV samples of the EPIZONE reference RNA panel. Cq-values of undiluted and 100-fold diluted sample RNAs are shown.**

| **CSFV sample** | **NS5A system** | | **CSF1 system** | |
|---|---|---|---|---|
| | Undiluted | diluted 1:100 | Undiluted | diluted 1:100 |
| C strain | 25.9 | 33.4 | 28.3 | 38.0 |
| Eystrup91 | 29.4 | 38.8 | 27.7 | 38.6 |
| Alfort187 | 29.5 | 37.7 | 27.3 | 37.4 |
| Koslov1128 | 28.7 | 36.0 | 27.2 | Negative |
| Brescia | 27.6 | 36.3 | 30.5 | 36.9 |
| Schweiz II | 29-8 | 38.9 | 28.4 | 38.7 |
| Pader | 28 | 36.6 | 27.2 | Negative |
| Bergen | 29.4 | 36.2 | 28 | 41.1 |
| D4886/82/Ro | 28.1 | 36.6 | 28.1 | 39.6 |
| Uelzen | 27.5 | 37.8 | 28.8 | 36.8 |
| Spante | 27 | 34.7 | 27.8 | 37.2 |
| Congenital Tremor | 25.6 | 33.2 | 30.4 | 35.5 |
| Kanagawa | 28.1 | 36.1 | 27.4 | 36.7 |

**Table 4: Twenty different European CSFV RNA samples of genotype 2.3 were tested with the new CSFV specific NS5A real-time RT-PCR system and compared to the CSF 1 system. RNA samples were obtained by RNA extraction from cell culture supernatant with QIAamp® Viral RNA Mini Kit and diluted 1000-folk in RSB50 buffer. Cq-values are average values of samples tested in duplicate.**

| **CSFV Genotype 2.3 sample** | **NS5A system** | | **CSF 1 system** | |
|---|---|---|---|---|
| | Sample original | Sample 1:1000 diluted | Sample original | Sample 1:1000 diluted |
| CSF 1027 | 17.8 | 27.8 | 18.3 | 28.0 |
| CSF 1024 | 17.8 | 27.9 | 18.8 | 29.1 |
| CSF 0867 | 18.6 | 28.4 | 19.6 | 29.7 |
| CSF 0866 | 25.1 | 34.9 | 23.5 | 33.8 |
| CSF 0864 | 20.3 | 29.6 | 19.1 | 29.1 |
| CSF 1015 | 18.3 | 28.1 | 17.9 | 27-9 |
| CSF 0852 | 18.9 | 28.4 | 17.9 | 27.8 |
| CSF 0854 | 18.5 | 28.1 | 19.1 | 29.2 |
| CSF 0848 | 31.3 | 40.3 | 29.9 | 37.9/negative |
| CSF 0847 | 20.9 | 30.8 | 20.6 | 30.6 |
| CSF 0840 | 23.6 | 33.8 | 23.0 | 33.2 |
| CSF 0838 | 26.7 | 36.8 | 27.0 | 38.1 |
| CSF 0822 | 18.6 | 28.3 | 19.4 | 29.4 |
| CSF 0821 | 18.1 | 27.8 | 19.5 | 29.9 |
| CSF 0817 | 18.8 | 28.6 | 20.6 | 30.9 |
| CSF 0807 | 22.2 | 32.2 | 20.2 | 30.4 |
| CSF 0804 | 20.1 | 30.1 | 19.8 | 30.0 |
| CSF 0085 | 18.7 | 28.7 | 20.1 | 30.9 |
| CSF 0737 | 18.6 | 28.7 | 19.9 | 30.6 |
| CSF 0727 | 20.2 | 30.1 | 21.6 | 31.4 |

**Table 5: Real-time RT-PCR results in the NS5A according to the present invention and the Pan-Pesti systems for Non-CSFV Pestivirus strains of the EPIZONE reference RNA panel. Cq-values are shown.**

| Pestivirus genotype | Pestivirus strain | NS5A system | Pan-Pesti system Hoffmann et al. 2006 |
|---|---|---|---|
| BDV | Moredun | neg | 34,16 |
| BDV | Rudolph | neg | 29,09 |
| BDV | Gifhorn | neg | 32,01 |
| BDV | Isard | neg | 32,01 |
| BVDV-1 | NADL | neg | 32,24 |
| BVDV-1 | Paplitz | neg | 29,19 |
| BVDV-1 | PI809 | neg | 30,55 |
| BVDV-1 | NC3807-1251/1 | neg | 30,5 |
| BVDV-1 | Egbert | neg | 34,64 |
| BVDV-1 | B0806-17 | neg | 30,14 |
| BVDV-1 | B0807-3 | neg | 29,7 |
| BVDV-1 | NC3807-8757 | neg | 31,08 |
| BVDV-2 | 8644 | neg | 32,8 |
| BVDV-2 | Bure | neg | 35,04 |
| BVDV-2 | Walter | neg | 31,78 |
| BVDV-2 | PO1600 | neg | 30,34 |
| Pestivirus | Hobi | neg | 33,57 |
| Pestivirus | Giraffe H138 | neg | 36,19 |

### Figure legends:

**Figure 1****:** Overview of the CSFV genome with the expressed proteins and a scale indicating the respective nucleotide position in the genome. The black arrows and ciphers show the approximate genome positions tested during development of the new CSFV specific real-time RT-PCR system.
**Figure 2****:** Clustalw2/BioEdit multi-alignment of different CSFV genomes from the NCBI database and newly sequenced CSFV isolates. The numbering of the nucleotides refers to the whole genome sequence of the AF326963 "Eystrup" strain (SEQ ID NO. 1).
**Figure 3****:** a) Amplification plot of a tenfold dilution series of the newly developed NS5A RNA standard tested with the CSFV specific NS5A real-time RT-PCR system. The numbers within the curves indicate the initial quantity (copy number) of CSFV genomes added to the reaction. b) Standard curve graph of the RNA standard from the NS5A specific real-time RT-PCR.

## Claims

1. A method for determining the presence of a classical swine fewer virus (CSFV) in a subject comprising the following step:
- detection of the NS5A region of at least one classical swine fewer virus (CSFV) genome in a sample derived from said subject;
wherein the detection of the NS5A region of the at least one classical swine fewer virus (CSFV) genome is attributed to the presence of a classical swine fewer virus (CSFV) in said subject.

2. The method according to claim 1, wherein the detection of the NS5A region comprises the use of a nucleic acid probe hybridizing to the NS5A region of at least one classical swine fewer virus (CSFV) genome and/or to a nucleic acid complementary to the at least one NS5A region of the at least one classical swine fewer virus (CSFV) genome.

3. The method according to claim 2, wherein the nucleic acid probe hybridizes to all sequences of the group consisting of SEQ ID NOs 8, 9, 10, 11, 12, 13, 14, 15, 16, 17, 18, 19, 20, 21, 22, 23, 2,4, 25, 26, 27, 28 ,29, 30, 31, 32, 33, 34, 35, 36, 37, 38, 39, 40, and 41, or fragments thereof.

4. The method according to claims 2 or 3, wherein the nucleic acid probe comprises at least one modification to increase the stability of the probe-target hybrid.

5. The method according to any one of claims 2 to 4, wherein the nucleic acid probe comprises at least at least 4 modifications to increase the stability of the probe-target hybrid, and wherein said modifications are locked nucleic acids (LNA).

6. The method according to any one of claims 2 to 5, wherein the nucleic acid probe comprises the sequence of SEQ ID NO. 2.

7. The method according to any one of claims 1 to 6, wherein prior to the detection of the NS5A region of the at least one classical swine fewer virus (CSFV) genome said genome of the at least one swine fewer virus (CSFV) or parts thereof is amplified.

8. The method according to claim 7, wherein the amplification product comprises the sequence according to SEQ ID NO. 2.

9. The method according to claims 7 or 8, wherein the amplification is performed using at least one CSFV amplification primer, wherein said at least one CSFV amplification primer comprises a 3'-end consisting of at least the last 6 nucleotides according to the sequence of SEQ ID NO. 3 or SEQ ID NO. 4.

10. The method according to any one of claims 1 to 9, wherein the amplification and/or detection of the NS5A region are performed by real-time RT-PCR.

11. A kit for determining the presence of a classical swine fewer virus (CSFV) in a subject comprising:
- a nucleic acid probe, wherein the nucleic acid probe is capable of hybridizing to the NS5A region of the genome of at least one classical swine fewer virus (CSFV) and/or to a nucleic acid complementary to the NS5A region of the genome of at least one classical swine fewer virus (CSFV), and/or at least one primer for sequencing of the NS5A region of the at least one classical swine fewer virus (CSFV) or parts thereof.

12. The kit according to claim 11, further comprising at least one CSFV amplification primers for amplifying the genome of at least one classical swine fewer virus (CSFV) or parts thereof, wherein the amplified part of the genome of said least one classical swine fewer virus (CSFV) comprises the sequence of SEQ ID NO. 2.

13. A nucleic acid of 16 to 40 nucleotides in lengths, wherein the nucleic acid comprises the sequence according to SEQ ID NO. 2.

14. A nucleic acid comprising a 3'-end consisting of at least the last 6 nucleotides according to the sequence of SEQ ID NO. 3 or SEQ ID NO. 4.

15. Use of a kit according to claims 11 or 12 or a nucleic acid according to claims 13 or 14 in a method according to any one of claims 1 to 10.


SEQUENCE LISTING

<110>  Labor Diagnostik GmbH Leipzig

<120>  Method for specific detection of classical swine fever virus

<130>  S1013 EP BLN

<160>  43

<170>  PatentIn version 3.5

<210>  1
<211>  12301
<212>  DNA
<213>  pestivirus type 2

<220>
<221>  NS5A
<222>  (8423)..(9913)

<300>
<308>  AF326963
<309>  2008-12-08
<313>  (1)..(12301)

<400>  1
gtatacgagg ttagttcatt ctcgtatgca tgattggaca aatcaaaatt tcaatttggt      60

tcagggcctc cctccagcga cggccgaact gggctagcca tgcccacagt aggactagca     120

aacggaggga ctagccgtag tggcgagctc cctgggtggt ctaagtcctg agtacaggac     180

agtcgtcagt agttcgacgt gagcagaagc ccacctcgag atgctatgtg gacgagggca     240

tgcccaagac acaccttaac cctagcgggg gtcgctaggg tgaaatcaca ccacgtgatg     300

ggagtacgac ctgatagggc gctgcagagg cccactatta ggctagtata aaaatctctg     360

ctgtacatgg cacatggagt tgaatcattt tgaactttta tacaaaacaa acaaacaaaa     420

accaatggga gtggaggaac cggtatacga tgccacgggg aggccattgt ttggagaccc     480

gagtgaggta cacccacaat caacactgaa gctaccacat gataggggga gaggtaacat     540

caaaacaaca ctgaagaacc tacctaggaa aggcgactgc aggagtggca accatctagg     600

cccggttagt gggatatatg taaagcccgg ccctgtcttt tatcaggact acatgggccc     660

ggtctaccat agagcccctc tagagttttt taacgaagcg cagttttgcg aggtgaccaa     720

aaggataggt agggtgacag gtagtgacgg aaagctttac catatatatg tgtgcatcga     780

tggttgcata ctgctgaagc tagccaagag ggacgagcca agaaccctga agtggattag     840

aaatttcacc gactgtccat tgtgggttac cagttgctct gatgatggcg caagtggaag     900

taaagagaag aagccagata ggatcaacaa aggcaaatta aaaatagccc caaaagagca     960

tgagaaggac agcagaacta agccacctga cgctacgatt gtagtggaag gagtaaaata    1020

ccaggttaaa aagaagggta aagttaaagg aaagagtacc caagacggcc tgtaccacaa    1080

caagaataaa ccaccagaat ctaggaagaa attagaaaaa gccctattgg catgggcggt    1140

aatagcaatt atgttgtacc aaccagttga agccgaaaat ataactcaat ggaacctgag    1200

tgacaacggc actaatggta tccagcatgc tatgtacctt agaggggtta gcaggagctt 1260
gcatgggatc tggccggaaa aaatatgcaa aggagtcccc acctacctgg ccacagacac 1320
ggaactgaaa gaaatacagg gaatgatgga tgccagcgag gggacaaact atacgtgctg 1380
taagttacag agacatgaat ggaacaaaca tggatggtgt aactggtaca atatagaccc 1440
ctggatacag ttgatgaata gaacccaagc aaacttggca gaaggccctc cggccaagga 1500
gtgcgctgtg acttgcaggt acgataaaga tgctgacatc aacgtggtca cccaggccag 1560
aaacaggcca acaaccctga ccggttgcaa gaaaggaaaa aatttttctt ttgcgggtac 1620
agttatagag ggcccatgta atttcaatgt ttccgtggag gatatcttgt atggggatca 1680
tgagtgcggc agtttgcttc aggacacggc tctgtaccta gtggatggaa tgaccaacac 1740
tatagagaat gccagacagg gagcagcgag ggtaacatct tggctcggga ggcaactcag 1800
cactgccggg aagaggttgg agggtagaag caaaacctgg tttggtgcct atgccctatc 1860
gccttactgt aatgtaacaa gcaaaatagg gtacatatgg tacactaaca actgcacccc 1920
ggcttgcctc cccaaaaata caaagataat aggccccgga aaatttgaca ctaacgcgga 1980
agacggaaag attctccatg agatgggggg tcacctatca gaatttctgc tgctctctct 2040
ggttgttctg tctgacttcg cccctgaaac agccagcgcg ttatacctca ttttgcacta 2100
catgattcct caatcccatg aagaacctga aggctgcgac acaaaccagc tgaatctaac 2160
agtggaactc aggactgaag acgtaatacc gtcatcagtc tggaatgttg gcaaatatgt 2220
gtgtgttaga ccagactggt ggccatatga aaccaaggtg gctttgttat ttgaagaggc 2280
aggacaggtc gtaaaattag ccttacgagc gctgagggat ttaaccaggg tctggaatag 2340
cgcatcaacc acggcattcc tcatctgctt gataaaagta ttaagaggac agatcgtgca 2400
aggtgtgata tggctgctac tagtaactgg ggcacaaggc cggctagcct gcaaggaaga 2460
ttacaggtac gcaatatcat cgaccaatga gatagggcta ctcggggccg aaggtctcac 2520
caccacctgg aaagaataca accacgattt gcaactgaat gacgggaccg ttaaggccat 2580
ttgcgtggca ggttccttta aagtcatagc acttaatgtg gtcagtagga ggtatttggc 2640
atcattgcat aaggaggctt cactcacttc cgtgacattt gagctcctgt tcgacgggac 2700
caacccatca actgaggaaa tgggagatga cttcgggttc gggctgtgcc cgttcgatac 2760
gagtcctgtt gtcaagggaa agtacaatac aaccttgttg aacggtagtg ctttctatct 2820
tgtctgccca atagggtgga cgggtgtcat agagtgcaca gcagtgagcc caacaactct 2880
gagaacagaa gtggtaaaga ccttcaggag agacaagccc tttccgcaca gaatggattg 2940
tgcgaccacc acagtggaaa atggagattt attctactgt aagttggggg gcaactggac 3000
atgtgtgaaa ggtgaaccag tggtctacac gggggggcta gtaaaacaat gcagatggtg 3060
tggcttcgac ttcaatgagc ccgacggact cccgcactac cccataggta agtgcatctt 3120
ggtaaatgag acaggttaca gaatagtaga ttcaacggac tgtaacagag atggcgttgt 3180
aatcagcaca gatgggagtc atgagtgctt gatcggtaac acaactgtca aggtgcatgc 3240

atcagatgaa agactgggcc ctatgccatg cagacccaaa gagattgtct ctagtgcagg 3300
acctgtaagg aaaacttcct gtacattcaa ctacgcaaaa actttgaaga acaagtacta 3360
tgagcccagg gacagctact tccagcaata tatgcttaag ggcgagtatc agtactggtt 3420
tgacctggac gtgactgacc gccactcaga ttacttcgca gaatttgtcg tcttggtagt 3480
ggtagcactg ttaggaggaa gatatgtcct gtggctaata gtgacctaca tagttctaac 3540
agaacaactc gccgctggtt taccattggg ccagggtgag gtagtgttga tagggaactt 3600
aattacccac acagacattg aggtcgtagt atatttctta ctactctatt tggtcatgag 3660
ggatgagcct ataaagaaat ggatactgct gctgttccat gctatgacta acaatccagt 3720
caagaccata acagtggcat tgcttatggt tagtggggtt gccaagggtg gaaagataga 3780
cggcggttgg cagcggctgc cagagaccag ctttgacatc caactcgcgc tgacagttat 3840
agtagtcgct gtgatgttac tggcaaagag agatccaact actgtcccct tggttataac 3900
agtggcaacc ctgagaacgg ctaagatgac taatggactt agcacggata tagccatagc 3960
tacagtgtca acagcgttgc taacctggac ctacattagt gactattata gatacaagac 4020
ttggctacag taccttatta gcacagtgac aggtatcttc ttaataaggg tactgaaggg 4080
aataggtgag ttggatttac acactccaac cttgccatct tacagacccc tcttcttcat 4140
tctcgtgtac ctcatttcca ctgcagtggt aacaagatgg aatctggaca tagccggatt 4200
gctgttgcag tgtgtcccaa cccttttgat ggtttttacg atgtgggcag acattctcac 4260
cctgatcctc atactgccca cttacgagct aacaaaacta tattacctca aggaagtgaa 4320
gactggggca gaaaagggct ggttatggaa gaccaacttc aagagggtaa acgacatata 4380
cgaagttgac caatctggtg aaggggttta ccttttcccg tcaaaacaaa agacaagttc 4440
aataacaggt accatgttgc cattgatcaa agccatactc atcagctgca tcagtaataa 4500
gtggcagttc atatatctat tgtacttgat atttgaagtg tcttactacc tccacaagaa 4560
gatcatagat gaaatagcag gagggaccaa cttcatctca agacttgtag ccgctttgat 4620
cgaagccaat tgggcctttg acaacgaaga agttaggggt ttaaagaagt tcttcctgtt 4680
gtctagtagg gttaaagaac tgatcatcaa acacaaagtg aggaatgaag taatggtcca 4740
ctggtttggt gacgaagagg tttatgggat gccaaagttg gttggcttag tcaaggcagc 4800
aacattgagt aaaaataaac attgtatttt gtgcaccgtc tgtgaagaca gagagtggag 4860
aggagaaacc tgcccaaaat gcgggcgttt tgggccacca atgacctgtg gtatgaccct 4920
agccgacttt gaagaaaaac attataagag gatctttttt agagaggatc aatcagaagg 4980
gccggttaga gaggagtacg cagggtatct gcaatataga gccagagggc aattattcct 5040
gaggaatctc ccggtgctag caacaaaagt caagatgctc ctggtcggaa atcttgggac 5100
ggaggtggga gacttggaac accttggctg ggtccttagg gggcctgccg tttgcaagaa 5160
ggttaccgaa catgagaaat gcaccacatc cataatggac aaattgactg cttttttcgg 5220
tgttatgcca aggggcacca cacctagagc ccctgtgaga ttccccacct ctctcttaaa 5280

gataagaagg gggttggaaa ctggctgggc gtacacacac caaggtggca ttagttcagt 5340
ggaccatgtc acttgtggga aagacttgct ggtatgtgac actatgggcc ggacaagggt 5400
cgtttgccaa tcaaataata agatgacaga tgagtctgag tatggagtta aaactgactc 5460
cggatgcccg gaaggagcta ggtgttatgt gttcaaccca gaggcagtta acatatcagg 5520
gactaaagga gccatggtcc acttacaaaa aactggagga gaattcacct gtgtgacagc 5580
atcaggaact ccggccttct ttgatctcaa gaacctcaaa ggctggtcag ggctaccgat 5640
atttgaggca tcaagtggaa gggtagtcgg cagggtcaag gtcgggaaga atgaggactc 5700
taaaccaacc aagcttatga gtggaataca aacagtctcc aaaagtacca cagacttgac 5760
agaaatggta aagaaaataa cgaccatgaa caggggagaa ttcagacaaa taacccttgc 5820
tacaggtgcc ggaaaaacca cggaactccc taggtcagtc atagaagaga tagggaggca 5880
taagagagtc ttggtcttga tccctctgag ggcggcagca gagtcagtat accaatatat 5940
gagacaaaaa catccaagca tcgcatttaa cctgaggata ggggagatga aggaagggga 6000
catggccaca gggataacct atgcttcata cggttacttc tgtcagatgc cacaacctaa 6060
gttgcgagcc gcgatggttg agtactcctt catatttctt gacgagtacc actgtgccac 6120
cccagaacaa ttggccatca tgggaaagat ccacagattt tcagagaacc tgcgggtagt 6180
agccatgacc gcaacaccag caggcacagt aacaaccaca gggcagaaac accctataga 6240
agaattcata gccccagaag tgatgaaagg ggaagactta ggctcagagt acttggacat 6300
tgctggacta aagataccag tagaggagat gaagagcaac atgctggttt ttgtgcccac 6360
taggaacatg gcggtggaga cagcaaagaa attgaaagct aagggttaca actcaggcta 6420
ctattatagt ggagaggatc catctaacct gagggtggta acgtcgcagt ccccgtacgt 6480
ggtggtggca accaacgcga tagaatcagg tgttactctc ccggacttgg atgtggttgt 6540
cgatacaggg cttaagtgtg aaaagagaat acggctgtca cctaagatgc ccttcatagt 6600
gacgggcctg aagagaatgg ctgtcacgat tggggaacaa gcccagagaa gggggagagt 6660
tgggagagta aagcctggga gatactacag gagtcaagaa actcccgttg gttctaaaga 6720
ttaccattat gatctactgc aagcacagag gtacggtatt gaagatggga taaacatcac 6780
caaatccttt agagagatga actatgattg gagcctttat gaggaggaca gtctgatgat 6840
tacacaattg gaaatcctca ataatttgtt gatatcagaa gaactaccga tggcagtaaa 6900
aaatataatg gccaggactg accacccaga accaattcag ctggcgtaca acagctacga 6960
aacacaagtg ccagtgctat tcccaaaaat aaagaatgga gaggtgactg atagttacga 7020
taactatacc ttcctcaacg caagaaaatt gggggatgat gtaccccctt acgtgtatgc 7080
cacagaggat gaggacttag cggtagagct actgggctta gactggccag accctggaaa 7140
ccaaggaacc gtagaggctg gcagagcact aaaacaagta gttggtctat caacagctga 7200
gaatgccctg ttagtagcct tattcggcta tgtaggatat caggcacttt caaagaggca 7260
tataccagta gtcacagata tatattcaat tgaagatcac aggttggaag acaccacaca 7320

cctacagtac gccccaaatg ctatcaagac ggaggggaag gagacagaat tgaaggagct 7380
agcccagggg gatgtgcaga gatgtgtgga agctatgacc aattatgcaa gagagggtat 7440
ccaattcatg aagtctcagg cactgaaggt gaaagaaacc cccacttaca aagagacaat 7500
gaacactgtg actgactatg taaagaaatt catggaggcg ctggcagaca gtaaagaaga 7560
catcttaaga tatgggttgt gggggacgca cacagcctta tataagagca tcagtgccag 7620
gcttgggagt gagactgcgt tcgctaccct ggtcgtgaag tggctggcat ttggggggga 7680
atcaatagca gaccatgtca aacaagcggc cacagacttg gtcgtctact atatcatcaa 7740
cagacctcag ttcccaggag acacagagac acaacaggaa ggaaggaaat ttgtggccag 7800
cctactggtc tcagctctag ctacttacac atacaaaagc tggaattaca ataatctgtc 7860
caagatagtt gaaccggctt tggccactct gccctatgcc gccacagctc tcaaactatt 7920
cgcccccact cgattggaga gcgttgtcat attgagtacc gcaatctaca agacctacct 7980
atcaatcagg cgcggaaaaa gcgatggttt gctaggcaca ggggttagtg cggctatgga 8040
gatcatgtca caaaatccag tatccgtggg catagcagtc atgctagggg taggggccgt 8100
ggcagcccac aatgcaatcg aggccagtga gcagaagaga acactactca tgaaagtttt 8160
tgtaaagaac ttcttggacc aagcagccac tgatgaatta gtcaaggaga gtcctgagaa 8220
aataataatg gctttgtttg aagcagtgca gacagtcggt aaccctctta gactagtata 8280
ccacctttat ggagttttct ataaggggtg ggaggcaaaa gagttggccc aaaggacagc 8340
cggtaggaac cttttcactt tgataatgtt cgaggctgtg gaactactgg gagtagatag 8400
tgaaggaaag atccgccagc tatcaagtaa ttacatacta gagctcctgt ataagttccg 8460
tgacagtatc aagtctagcg tgagggagat ggcaatcagc tgggcccctg cccctttcag 8520
ctgtgattgg acaccgacgg atgacagaat agggctcccc caagacaatt tcctccaagt 8580
ggagacgaaa tgcccctgtg gttacaagat gaaggcagtt aagaattgtg ctggagagct 8640
gagactcttg gaggaggaag gctcatttct ctgcagaaat aaattcggga gaggttcacg 8700
gaactacagg gtgacaaaat actatgatga caatctatca gaaataaagc cagtgataag 8760
aatggaaggg catgtggaac tctactacaa gggagccacc atcaaactgg acttcaacaa 8820
cagtaaaaca atactggcaa ccgataaatg ggagattgat cactccactc tggtcagggt 8880
gctcaagagg cacacagggg ctggatatca tggggcatac ctgggcgaga aaccgaacta 8940
caaacatctg atagagaggg actgtgcaac catcaccaaa gataaggttt gttttctcaa 9000
aatgaagaga gggtgtgcat ttacttatga cttatccctt cacaacctta cccgactgat 9060
tgaattggta cacaagaata acttggaaga caaagagatt cctgctgtta cggttacaac 9120
ctggctggct tacacgtttg taaatgaaga tatagggacc ataaaaccag ccttcgggga 9180
gaaagtaaca ccggagatgc aggaggaaat aaccttgcag cctgctgtag tggtggatac 9240
aactgacgtg accgtgactg tggtagggga agcccctact atgactacag gggagactcc 9300
gacagcgttc accagctcag gttcagaccc gaaaggccaa caagttttaa aactgggggt 9360

aggtgaaggc caataccccg ggactaatcc acagagggca agcctgcacg aagccataca 9420
aggtgcagat gagagaccct cggtgctgat attagggtct gataaagcca cctctaatag 9480
agtgaaaact gcaaagaatg taaaggtata cagaggcagg gacccactag aagtgagaga 9540
tatgatgagg aggggaaaga tcctggtcat agccctgtct agggttgata atgctctatt 9600
gaaatttgtt gactacaaag gcacctttct aactagagag accctagagg cattaagttt 9660
gggtaggcct aaaaagaaaa acataaccaa ggcagaagcg cagtggttgc tgtgcctcga 9720
agaccaaatg gaagagctac ccgattggtt cgcagccggg gaacccattt ttctagaggc 9780
taacattaaa catgacaggt accatctggt gggggatata gctaatatca aggaaaaagc 9840
caaacagttg ggagctacag actccacaaa gatatctaag gaggttggtg caaaagtgta 9900
ttctatgaaa ctgagtaatt gggtgatgca agaagaaaat aaacagggca acctgacccc 9960
cttgtttgaa gagctcctgc aacagtgtcc acccggaggc cagaacaaaa ctgcacatat 10020
ggtctctgct taccaactag ctcaagggaa ctggatgcca accagctgcc atgtttttat 10080
ggggaccata tctgccagga ggaccaagac ccatccatat gaagcatacg tcaagttaag 10140
ggagttggta gaggaacaca agatgaaaac attgtgtcct ggatcaagcc tgggtaagca 10200
caacgaatgg ataattggta aaatcaaata ccagggaaac ctgaggacca aacacatgtt 10260
gaaccccggc aaggtggcag agcaactgtg cagagaggga cacagacaca atgtgtataa 10320
caagacaata ggctcagtaa tgacagctac tggtatcagg ttggagaagt tgcccgtggt 10380
tagggcccag acagacacaa ccaacttcca ccaagcaata agggataaga tagacaagga 10440
agagaaccta caaaccccgg gtttacataa gaaactaatg gaagttttca atgcattgaa 10500
acgacccgag ttagagtcct cctacgatgc cgtggaatgg gaggaactgg agagaggaat 10560
aaacaggaag ggtgctgctg gtttctttga acgcaaaaat ataggggaaa tattggattc 10620
agagaaaaac aaagtcgaag agattattga caatctgaaa aaaggcagaa acatcaaata 10680
ctatgaaacc gcgatcccaa agaatgagaa gagggacgtc aatgatgact ggactgctgg 10740
tgacttcgtg gaagagaaga aacccagagt catacaatac cctgaagcaa aaacaaggct 10800
ggccatcacc aaggtgatgt ataagtgggt gaagcagaag ccagtagtta tacccgggta 10860
tgaagggaag acacctctat tccaaatttt tgacaaagta aagaaggaat gggatcaatt 10920
ccaaaatcca gtggcagtga gttttgacac taaggcgtgg gacacccagg taaccacaaa 10980
agatttggag ttgataaagg acatacaaaa gtactatttc aagaagaaat ggcataaatt 11040
tattgacacc ctgaccatgc acatgtcaga agtacccgta atcagtgctg atggggaagt 11100
atacataagg aaagggcaaa gaggcagtgg acaacctgac acaagcgcag gcaatagcat 11160
gctaaatgtg ttaacaatga tttacgcctt ctgcgaggcc acgggagtac cctacaagag 11220
cttcgacagg gtggcaaaaa ttcatgtgtg tggggatgat ggtttcctga tcacagaaag 11280
agctctcggt gagaaattcg cgagtaaggg agtccagatc ctatatgaag ctgggaagcc 11340
ccagaagatc actgaagggg acaagatgaa agtggcctac caatttgatg atattgagtt 11400

ttgctcccat acaccaatac aagtaaggtg gtcagataac acttctagtt acatgccggg 11460

gagaaataca accacaatcc tggctaaaat ggccacaagg ttagattcca gtggtgagag 11520

gggtaccata gcatatgaga aagcagtagc attcagcttc ctgctgatgt actcctggaa 11580

cccactaatc agaaggatct gcttactggt gctatcaact gaactgcaag tgaaaccagg 11640

gaagtcaacc acttactact atgaagggga cccgatatct gcctacaagg aagtcatcgg 11700

ccacaatctt tttgatctta agagaacaag cttcgagaag ctggccaagt taaatctcag 11760

catgtcagta ctcggagcct ggactagaca caccagtaaa agactactac aagactgtgt 11820

caatgtgggt gttaaagagg gcaactggct agttaatgca gatagactag taagtagcaa 11880

gactggaaat aggtacatac ccggagaggg ccacaccctg caagggagac attatgaaga 11940

actggtgttg gcaagaaaac agatcaacaa ctttcaaggg accgacaggt acaatctagg 12000

cccaatagtc aatatggtgt taaggaggct gagagtcatg atgatgaccc tgatagggag 12060

aggggtatga gcgcgggtaa cccgggatct ggacccgcca gtagaaccct gttgtagata 12120

acactaattt ttttttattt atttagatat tactatttat ttatttattt atttattgaa 12180

tgagtaagaa ctggtacaaa ctacctcaag ttaccacact acactcattt ttaacagcac 12240

tttagctgga aggaaaattc ctgacgtcca cagttggact aaggtaattt cctaacggcc 12300

c 12301

<210> 2
<211> 16
<212> DNA
<213> Artificial Sequence

<220>
<223> nucleic acid probe

<400> 2
tgataytrgg gtcyga 16

<210> 3
<211> 32
<212> DNA
<213> Artificial Sequence

<220>
<223> CSFV amplification primer (forward)

<400> 3
aataaatcat aagcctgcwy gaagcyatac aa 32

<210> 4
<211> 27
<212> DNA
<213> Artificial Sequence

<220>
<223> CSFV amplification primer (reverse)

<400> 4
cttyacattc tthrcrgtyt tyacyct 27

<210> 5
<211> 18
<212> DNA
<213> Artificial Sequence

<220>
<223> primer for amplification of beta-actin reference nucleic acid

<400> 5
btccttcctg ggcatgga 18

<210> 6
<211> 19
<212> DNA
<213> Artificial Sequence

<220>
<223> reverse primer for amplification of beta-actin reference nucleic
acid

<400> 6
grggsgcgat gatcttgat 19

<210> 7
<211> 29
<212> DNA
<213> Artificial Sequence

<220>
<223> nucleic acid probe for beta-actin reference nucleic acid

<400> 7
tccatcatga agtgygacgt sgacatccg 29

<210> 8
<211> 120
<212> DNA
<213> pestivirus type 2

<400> 8
tcagcagagg gcaagcttgc ttgaagctat acaaggtgtg gatgaaaggc cctcggtact 60

gatattgggg tctgataagg ccacctccaa tagggtgaag accgcaaaga atgtgaagat 120

<210> 9
<211> 120
<212> DNA
<213> pestivirus type 2

<400> 9
tccacagagg gcaagcctgc acgaagccat acaaggtgct gatgagaggc cctcggtgct 60

gatattgggg tctgataaag ccacctctaa tagagtaaaa actgcaaaga atgtaaaggt 120

<210> 10
<211> 120
<212> DNA
<213> pestivirus type 2

<400> 10
tccacagagg gcaagcctgc acgaagccat acaaggtgca gatgagaggc cctctgtgct 60

gatattgggg tctgataaag ccacctctaa tagagtgaaa actgcaaaga atgtaaaggt 120

<210> 11
<211> 120
<212> DNA
<213> pestivirus type 2

<400> 11
tccacagagg gcaagcctgc acgaagccat acaaggtgca gatgagaggc cctctgtgct 60

gatattgggg tctgataaag ccacctctaa tagagtgaaa actgcaaaga atgtaaaggt 120

<210> 12
<211> 120
<212> DNA
<213> pestivirus type 2

<400> 12
ccagcagagg gcaagtctgc acgaagccat acaaggggca gatgaaaggc cctcagtact 60

gatactgggg tctgataaag ccacctccaa tagagtaaaa acagcaaaaa atgtgaagat 120

<210> 13
<211> 120
<212> DNA
<213> pestivirus type 2

<400> 13
tccacagagg gcaagcctgc acgaagccat acaaggtgca gatgagaggc cctcggtgct 60

gatattgggg tctgataaag ccacctctaa tagagtgaag actgcaaaga atgtaaaggt 120

<210> 14
<211> 120
<212> DNA
<213> pestivirus type 2

<400> 14
tccacagagg gcaagcctgc acgaagccat acaaggtgca gatgagaggc cttcggtgct 60

gatattgggg tctgataaag ccacctctaa tagagtgaaa actgctaaga atgtaaaggt 120

<210> 15
<211> 120
<212> DNA
<213> pestivirus type 2

<400> 15
tccacagagg gcaagcctgc acgaagccat acaaggtgct gatgagaggc cctcggtgct 60

gatattgggg tctgataaag ccacctctaa tagagtaaaa actgcaaaga atgtaaaggt 120

<210> 16
<211> 120
<212> DNA
<213> pestivirus type 2

<400> 16
tcagcagaga gcaagcctgc tcgaagctat acaaggtgtg gatgaaaggc cctcggtact 60

gatactgggg tctgataagg ccacctccaa tagggtcaag accgcaaaga atgtgaagat 120

<210> 17
<211> 120
<212> DNA
<213> pestivirus type 2

<400> 17
tccacagagg gcaagcctgc acgaagccat acaaggtgca gatgagaggc cttcggtgct 60

gatattgggg tctgataaag ccacctctaa tagagtgaaa actgctaaga atgtaaaggt 120


<210> 18
<211> 120
<212> DNA
<213> pestivirus type 2

<400> 18
tccacagaga gcaagcctgc acgaagccat acaaaatgca gatgaaaggc cctcggtgct 60

gatattgggg tctgataaag ccacctctaa tagagtgaaa actgtgaaga atgtgaaggt 120


<210> 19
<211> 120
<212> DNA
<213> pestivirus type 2

<400> 19
tccacagaga gcaagcctgc acgaagccat acaaagcgca gatgaaaggc cctctgtgtt 60

gatattgggg tctgataaag ccacctctaa tagagtgaaa actgtaaaga atgtgaaggt 120


<210> 20
<211> 120
<212> DNA
<213> pestivirus type 2

<400> 20
tccacagaga gcaagcttgc tcgaagctat acaaggtggg gatgaaaggc cctcggtact 60

gatattgggg tctgataagg ccacctccaa tagggtgaag accgcaaaga atgtgaagat 120


<210> 21
<211> 120
<212> DNA
<213> pestivirus type 2

<400> 21
tccacagagg gcaagcctgc acgaagccat acaaggtgca gatgagaggc cctcggtgct 60

gatattgggg tctgataaag ccacctctaa tagagtgaag actgcaaaga atgtaaaggt 120


<210> 22
<211> 120
<212> DNA
<213> pestivirus type 2

<400> 22
tccacagagg gcaagcctgc acgaagccat acaaggtgca gatgagaggc cctcggtgct 60

gatattgggg tctgataaag ccacctctaa tagagtgaag actgcaaaga atgtaaaggt 120


<210> 23

<211>  120
<212>  DNA
<213>  pestivirus type 2

<400>  23
tccacagagg gcaagcctgc acgaagccat acaaggtgca gatgagagac cctcggtgct      60

gatattaggg tctgataaag ccacctctaa tagagtgaaa actgcaaaga atgtaaaggt      120


<210>  24
<211>  120
<212>  DNA
<213>  pestivirus type 2

<400>  24
tccacagagg gcaagcctgc acgaagccat acaaggtgca gatgagaggc cctcggtgct      60

gatattgggg tctgataaag ccacctctaa tagagtgaag actgcaaaga gtgtaaaggt      120


<210>  25
<211>  120
<212>  DNA
<213>  pestivirus type 2

<400>  25
tcagcagaga gcaagcttgc tcgaagctat acaaggtgtg gatgaaaggc cctcggtact      60

gatattgggg tctgataaag ccacctccaa cagggtgagg accgcaaaga atgtcaagat      120


<210>  26
<211>  120
<212>  DNA
<213>  pestivirus type 2

<400>  26
tcagcagaga gcaagcttgc tcgaagcaat acaaggtgtg gatgaaaggg cctcggtact      60

gatattgggg tctgataagg ccacctccaa cagggtgaag accgcaaaga atgtcaagat      120


<210>  27
<211>  120
<212>  DNA
<213>  pestivirus type 2

<400>  27
tccacagagg gcaagcctgc acgaagccat acaaggtgca gatgagaggc cctcggtgct      60

gatattgggg tctgataaag ccacctctaa tagagtgaag actgcaaaga atgtaaaggt      120


<210>  28
<211>  120
<212>  DNA
<213>  pestivirus type 2

<400>  28
tccacagagg gcaagcctgc acgaagccat acaaggtgca gatgagaggc cctctgtgct      60

gatattgggg tctgataaag ccacctctaa tagagtgaaa actgcaaaga atgtaaaggt      120


<210>  29
<211>  120
<212>  DNA

<213>  pestivirus type 2

<400>  29
tccacagagg gcaagcctgc acgaagccat acaaggtgca gatgagaggc cctctgtgct      60

gatattgggg tctgataaag ccacctctaa tagagtgaaa actgcaaaga atgtaaaggt      120


<210>  30
<211>  120
<212>  DNA
<213>  pestivirus type 2

<400>  30
tccacagaga gcaagcctgc acgaagccat acaaaatgca gatgaaaggc cctcggtgct      60

gatattgggg tctgataaag ccacctctaa tagagtgaaa actgtgaaga atgtgaaggt      120


<210>  31
<211>  120
<212>  DNA
<213>  pestivirus type 2

<400>  31
tccacagagg gcaagcctgc acgaagccat acaaggtgca gatgagaggc cctctgtgct      60

gatattgggg tctgataaag ccacctctaa tagagtgaaa actgcaaaga atgtaaaggt      120


<210>  32
<211>  120
<212>  DNA
<213>  pestivirus type 2

<400>  32
tccacagagg gcaagcctgc acgaagccat acaaggtgca gatgagaggc cctcggtgct      60

gatattgggg tctgataaag ccacctctaa tagagtgaag actgcaaaga atgtaaaggt      120


<210>  33
<211>  120
<212>  DNA
<213>  pestivirus type 2

<400>  33
tccacagagg gtaagtctgc acgaagccat acaaggtgcg gatgagaggc cctcagtact      60

gatattgggg tctgataaag ccacctctaa tagagtgaaa actgcaaaga atgtgaaggt      120


<210>  34
<211>  120
<212>  DNA
<213>  pestivirus type 2

<400>  34
tccacagagg gcaagcctgc acgaagccat acaaggtgct gatgagaggc cctcggtgct      60

gatattgggg tctgataaag ccacctctaa tagagtaaaa actgcaaaga atgtaaaggt      120


<210>  35
<211>  120
<212>  DNA
<213>  pestivirus type 2

<400>  35
tccacagagg gcaagcctgc acgaagccat acaaggtgca gatgagaggc cttcggtgct      60

gatattgggg tctgataaag ccacctctaa tagagtgaaa actgctaaga atgtaaaggt      120


<210>  36
<211>  120
<212>  DNA
<213>  pestivirus type 2

<400>  36
tccacagagg gcaagcctgc acgaagccat acaaggtgca gatgagaggc cctcggtgct      60

gatattgggg tctgataaag ccacctctaa tagagtgaag actgcaaaga atgtaaaggt      120


<210>  37
<211>  120
<212>  DNA
<213>  pestivirus type 2

<400>  37
tccacagaag gcaagcctgc acgaagccat acaaggtgca gatgagaggc cctctgtgct      60

gatattgggg tctgataaag ccacctctaa tagagtgaaa actgcaaaga atgtaaaggt      120


<210>  38
<211>  120
<212>  DNA
<213>  pestivirus type 2

<400>  38
tcagcagaga gctagcctgc tcgaagctat acaaggtgtg gatgaaaggc cctcggtact      60

gatactgggg tctgataagg ccacctctaa tagggtgaag accgcaaaga atgtgaagat      120


<210>  39
<211>  120
<212>  DNA
<213>  pestivirus type 2

<400>  39
tcagcagaga gctagcctgc tcgaagctat acaaggtgtg gatgaaaggc cctcggtact      60

gatactgggg tctgataagg ccacctccaa tagggtgaag accgcaaaga atgtgaagat      120


<210>  40
<211>  120
<212>  DNA
<213>  pestivirus type 2

<400>  40
tcagcagaga gctagcctgc tcgaagctat acaaggtgtg gatgaaaggc cctcggtact      60

gatactgggg tctgataagg ccacctctaa tagggtgaag accgcaaaga atgtgaagat      120


<210>  41
<211>  107
<212>  DNA
<213>  pestivirus type 2

<400>  41
aataaatcat aagcctgctc gaagcyatac aaagtgtgga tgaaaggccc tcggtactga      60

tattggggtc cgataaggcc acctccagta gggtgaagac cgcgaag 107

<210> 42
<211> 206
<212> DNA
<213> Sus scrofa

<400> 42
ctccttcctg ggcatggaat cctgcggcat ccacgaaact accttcaact ccatcatgaa 60

gtgcgacgtc gacatccgca aggacctcta cgccaacacg gtgctgtcgg gtggcaccac 120

catgtaccca ggcatcgccg acaggatgca gaaggagatc acggccctgg cgcccagcac 180

gatgaagatc aagatcatcg cgcctc 206

<210> 43
<211> 140
<212> DNA
<213> Artificial Sequence

<220>
<223> oLPC-CSF-NS5A [positive control nucleic acid for
CSF-9403.1-F-flap (forward primer), CSF 9501R (reverse primer)
and 9448-LNA-FAM (CSFV nucleic acid probe)]

<400> 43
agctgtaata cgactcacta tagggcgtga tgagcctgcw ygaagcyata caaacagccg 60

cacgagctct cgcagcatcc tgcctgatay trgggtcyga tatgcgcagr gtraaracyg 120

ydaagaatgt raagtgatgc 140